(19) Europäisches Patentamt
European Patent Office
Office européen des brevets

(11) EP 1 798 292 A1

(12) **EUROPEAN PATENT APPLICATION**

(43) Date of publication:
**20.06.2007 Bulletin 2007/25**

(51) Int Cl.:
***C12Q 1/68*** *(2006.01)*

(21) Application number: **05077921.4**

(22) Date of filing: **19.12.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **NUTRECO NEDERLAND B.V.**
**5831 JN Boxmeer (NL)**

(72) Inventors:
- **Rattink, Annemieke Paula**
  **3911 BB Rhenen (NL)**
- **Vereijken, Adrianus Lambertus Johannus**
  **5831 SB Boxmeer (NL)**

(74) Representative: **Winckels, Johannes Hubertus F. et al**
**Vereenigde**
**Johan de Wittlaan 7**
**2517 JR Den Haag (NL)**

Remarks:
The sequence listing, which is published as annex to the application documents, was filed after the date of filing. The applicant has declared that it does not include matter which goes beyond the content of the application as filed.

(54) **Methods for improving turkey meat production**

(57) The present invention relates to a method for selecting a bird of the species *Meleagris gallopavo* for having improved body weight gain characteristics, wherein said body weight gain is obtained by improved muscle deposition, said method comprising the steps of screening the genome of a population of candidate birds for the presence of at least one mutation in the myostatin gene associated with improved body weight gain characteristics.

EP 1 798 292 A1

## Description

FIELD OF THE INVENTION

The invention relates to turkey breeding, and in particular to methods for improving the growth and meat quantity and quality of birds in turkey growing.

BACKGROUND OF THE INVENTION

Poultry farming and in particular poultry growing for meat production are an integral part of agri-business. After pig meat, poultry meat is the most important meat in terms of worldwide production, and outranks pig meat as an export product. Poultry meat production keeps growing worldwide not in the least driven by expansion in China's domestic demand. The modern intensive system for poultry production provides relatively affordable meat for consumers.

Chickens, turkeys and other poultry grown for meat are mostly kept indoors. Chickens are grown for a period of around 40 days when they reach a weight of around 2 kg. Turkeys are grown to various ages up to 22 weeks, at which time males reach weights of up to around 22 kg. Within this growing period, the rate of gain in body weight of the birds is maximized. Preferably the body weight gain is essentially maximally associated with muscle deposition, and not with for instance fat deposition.

One way to improve the growth of the birds is to improve the growing system itself. Improving the birds' nutrition, health and climate, can all aid in improving growth performance and are an integral part of an improved farm management systems. Proper farm management can significantly reduce the number of days turkeys take to grow to their market weight. Another way to improve the growth of the birds is to improve the genetic constitution of the birds through breeding and selecting for strains having improved growth characteristics. The combination of selective breeding and improved farm management has dramatically increased the growth rate of turkeys over the last five decades.

Poultry breeding programmes are generally aimed at developing high-yielding, disease-resistant varieties by selecting those birds that exhibit the genetic characteristics or traits most desirable in the generation that is eventually grown for meat production. Generally a number of traits (around forty) is selected for, mostly based on phenotypic characteristics. For instance, robust heart, lungs and other organs, good leg strength, and resistance to disease are as important as body conformation, feed (conversion) efficiency and growth rate in the selection process. Selective breeding is now practised in all forms of livestock breeding. It essentially does not involve genetic engineering or genetic modification.

The generation of birds that is eventually grown for meat production are called the flock of commercials, or in short "commercials". In order to improve the genetic constitution of this production generation, their Parents, Grandparents and/or Great Grandparents, collectively called the multiplication stock, should already exhibit the desired genetic constitution. It is important that the (Great) Grandparent birds have genetic characteristics that allow for the Parent flocks to produce sufficient amounts of fertilized eggs from which chicks are hatched and reared for meat production. Consequently, the fertility or reproductive characteristics of the birds are equally important as their growth characteristics.

Eventually, selective breeding programmes are aimed at improving the pedigree, or elite flocks, whose offspring forms the multiplication stock. Only a few specialist primary breeding companies carry out selective breeding of commercial poultry strains. Due to the commercial interests, there is a continued need to improve the selective breeding methods. A very important aspect of modern selective breeding programmes is in maintaining sound and healthy birds with the proper genetic balance between reproduction in the Parent flocks and meat production in the commercials. It is however known that these two aspects are often incompatible, with high meat production often being associated with low reproduction. Thus, in selecting birds, these two aspects have to be observed.

Because phenotypic determination of both growth rate and reproductive traits can only be determined when the birds have reached a certain age, selection processes based on such determinations are expensive and time consuming, especially in turkey breeding. For this reason, there is a need for a method for selecting turkey birds having improved growth rates and/or higher weight at market age which can be applied at a very young age of the animals, preferably immediately after hatching of the birds, or even at the embryonic stage. Thus there is a need for a method which enables more accurate and more rapid selection for these desired traits in turkey.

SUMMARY OF THE INVENTION

The present inventors have now found a number of genetic markers that are associated with superior weight gain rates and muscle deposition characteristics in turkey.

In a first aspect, the present invention now relates to a method for selecting a bird of the species *Meleagris gallopavo* for having improved body weight gain characteristics, wherein said body weight gain is obtained by improved muscle deposition, said method comprising the steps of screening the genome of a population of candidate birds for the presence of at least one mutation in the myostatin gene associated with improved body weight gain characteristics. Preferably, said mutation is a single nucleotide polymorphisms (SNP) associated with improved body weight gain characteristics. Most preferably, said SNP is selected from the group consisting of SNPs numbered 1 to 23 as shown in Table 1.

The number of mutations in the myostatin gene associated with improved body weight gain characteristics, is preferably more than 2, more preferably more than 3 or 4, most preferably more than 5, 6, 7, 8, 9, 10, 11, 12, 13, 14, 15, 16, 17, 18, 19, 20, 21 or 22. The number of mutations in the gene of a single bird is herein termed the "number of mutations". The amount of birds in a population having mutations in the gene is herein termed the "frequency of mutations".

In another preferred embodiment of such a method, the body weight gain characteristics are higher growth rate and/or higher weight at market age.

In still another preferred embodiment of such a method, the said screening comprises obtaining a nucleic acid sample from each bird of said population of candidate birds, and screening said nucleic acid sample for the presence of at least one SNP in the myostatin gene.

In another aspect, the present invention relates to a method for producing a bird of the species *Meleagris gallopavo* comprising selecting a bird of the species *Meleagris gallopavo* according to a method as described in the first aspect, crossing said selected bird with a bird of a desirable turkey line and hatching the fertilized egg to produce the bird.

In a preferred embodiment of such a method, the desirable turkey line is an elite flock or a multiplication stock.

In another aspect, the present invention relates to a method of producing a turkey line having improved body weight gain characteristics, wherein said body weight gain is obtained by improved muscle deposition, said method comprising increasing the number of mutations in the myostatin gene in birds of said line, associated with improved body weight gain characteristics, as well as increasing the uniformity of that line for the presence of those mutations, i.e. increasing the frequency of mutations in the population of that line by increasing the number of birds that have mutations.

In a preferred embodiment of such a method, the mutations are SNPs selected from the group consisting of SNPs numbered 1 to 23 as shown in Table 1.

In another preferred embodiment of such a method, the body weight gain characteristics are higher growth rate and/or higher weight at market age.

In yet another preferred embodiment of such a method, the method comprising the method for producing a bird of the species *Meleagris gallopavo* as defined above.

In still another preferred embodiment of such a method, the turkey line is an elite flock, a multiplication stock or a commercial flock.

In still another preferred embodiment of such a method, the improved body weight gain characteristics are not associated with reduced egg production in female birds of multiplication stock.

In another aspect, the present invention relates to a bird of the species *Meleagris gallopavo* having improved body weight gain characteristics, wherein said body weight gain is obtained by improved muscle deposition, and wherein said bird has at least one, more preferably at least two, still more preferably at least 6 single nucleotide polymorphisms (SNP) in the myostatin gene associated with improved body weight gain characteristics.

In a preferred embodiment of such a bird, said at least one SNP is selected from the group consisting of SNPs numbered 1 to 23 as shown in Table 1.

In another preferred embodiment of such a bird, said body weight gain characteristics are higher growth rate and/or higher weight at market age.

In yet another preferred embodiment of such a bird, said SNPs are silent mutations.

In still another preferred embodiment of such a bird, said body weight gain characteristics are not associated with reduced egg production in female birds.

In another aspect, the present invention relates to a turkey line obtainable by a method of producing a turkey line having improved body weight gain characteristics according to the present invention.

In a preferred embodiment of such a turkey line said turkey line is an elite flock, a multiplication stock or a broiler flock.

In another aspect, the present invention relates to a turkey line exhibiting a frequency of mutations in the myostatin gene of at least 1, preferably at least 2, more preferably at least 3, 4 or 5, even more preferably 6, 7 or 8, mutations in the myostatin gene associated with improved body weight gain characteristics, most preferably mutations are the SNPs of Table 1. A turkey line of the present invention exhibits a uniformity, expressed as the percentage of birds of said flock having the desired mutation, of at least 80%, more preferably at least 90%, even more preferably at least 95%, still more preferably at least 98 or 99%, most preferably 100%. Preferably, said mutation is an SNP associated with increased muscle mass as defined herein.

SHORT DESCRIPTION OF THE DRAWINGS

Fig. 1 shows SEQ ID No. 1 representing the myostatin gene of Turkey as identified herein and indicating the mutations as listed in detail in Table 1 in bold type face by a corresponding IUPAC-IUB code for ambiguous nucleotides.

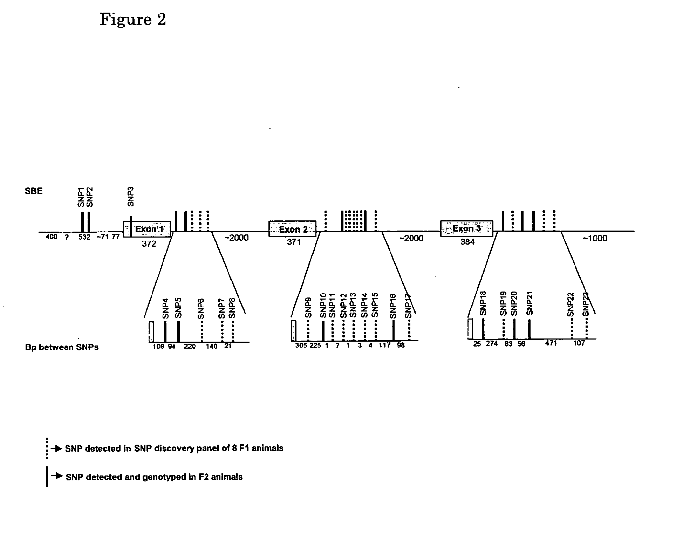

Fig. 2 shows an overview of the turkey GDF8 gene indicating introns and exons as well as the position of the various SNPs as identified herein.

Figure 3 shows SEQ ID No. 2 representing the myostatin gene of Turkey in the GenBank nucleic acid database collection under accession number AF019625.

DETAILED DESCRIPTION OF THE INVENTION

As used herein, the term "turkey" means a bird of the species *Meleagris gallopavo.*

The term "bird" as used herein refers to an individual turkey, unless the context specifically indicates otherwise.

A "breeder" is defined herein as a bird which is kept for fertile egg production.

A "commercial" is defined herein as a bird which is kept and raised for slaughter for meat yield.

The term "improved body weight gain characteristics", as used herein refers to those body weight gain characteristics obtained by improved muscle deposition. The "improved body weight gain characteristics" are brought about by mutations in the myostatin gene which generally have one or more of several phenotypic effects. The phenotypic effects may include for instance, an effect on live weight gain of the animal, i.e. increase of total body weight per unit of time; an effect on muscle growth of the animal, i.e. increase of number(hyperplasia) and/or size(hypertrophy) of muscle fibres; an effect on leanness of the animal, i.e. absolute or relative(to total body weight or to total muscle weight) reduction of deposition of fat into fat depots, intra-abdominally and/or subcutaneously, and/or intra-muscular fat; an effect on feed efficiency, i.e. reduced usage of feed intake per unit of total body weight increase; an effect on meat quality, i.e. better eating quality and/or better nutritional quality of turkey meats and turkey meat products; or an effect on reproductive characteristics of the bird, i.e. direct or indirect impact on expression of female and/or male pathways of reproductive physiology and expression of reproductive behaviour and reproductive success.

A "single nucleotide polymorphism" or "SNP" occurs at a polymorphic site occupied by a single nucleotide, which is the site of variation between allelic sequences. The site is usually preceded by and followed by highly conserved sequences of the allele (e.g., sequences that vary in less than 1/100 or 1/1000 members of the populations). A single nucleotide polymorphism usually arises due to substitution of one nucleotide for another at the polymorphic site. Single nucleotide polymorphisms can also arise from a deletion of a nucleotide or an insertion of a nucleotide relative to a reference allele.

As used herein, the term "allele (s)" means any of one or more alternative forms of a gene, all of which alleles relate to at least one trait or characteristic. In a diploid cell or organism, the two alleles of a given gene occupy corresponding loci on a pair of homologous chromosomes. An allele is also used to describe a given version of a polymorphism.

As used herein, the term "heterozygous" means a genetic condition existing when different alleles reside at corresponding loci on homologous chromosomes.

As used herein, the term "homozygous" means a genetic condition existing when identical alleles reside at corresponding loci on homologous chromosomes.

The term "homozygous presence of at least one allelic mutation" is referred herein as the presence of an identical mutation, more specifically specified by the SNP, at both alleles.

A "haplotype" is a set of alleles for closely spaced polymorphisms along a chromosome that tend to be inherited together. Alternatively a haplotype can be thought of as a combination of alleles of closely linked loci that are found in a single chromosomal interval and tend to be inherited together. An individual SNP allele can be used to define a given haplotype.

The term, "genotype," is used herein to mean a specific allele or alleles an individual carries at a given locus. It can also be used to describe a set of alleles for multiple loci.

The term "linkage disequilibrium" is used herein to refer to the relationship that is said to exist between an allele found at a single polymorphic site and alleles found at nearby polymorphisms if the presence of one allele is strongly predictive of the alleles present at the nearby polymorphic sites. Thus, the existence of linkage disequilibrium (LD) enables an allele of one polymorphic marker (SNP) to be used as a surrogate for a specific allele of another, i.e. the presence of one SNP may be indicative of the presence of another SNP.

As used herein, the term "breeding line" or "line" means a group of similar animals that by phenotypic and genotypic characteristics can be distinguished from other breeding lines within the same species of animal.

As used herein, the terms "line", "breeding line", "pure line" and "pure breeding line" are fully equivalent and are used interchangeably throughout this specification.

A "probe" or "nucleic acid probe" is a nucleic acid capable of binding to a target nucleic acid of complementary sequence through one or more types of chemical bonds, usually through complementary base pairing, usually through hydrogen bond formation, thus forming a duplex structure. A probe may include natural bases (i.e., A, G, C, or T) or modified bases (e.g., 7-deazaguanosine, inosine, etc.). A probe can be an oligonucleotide which is a single-stranded DNA. Oligonucleotide probes can be synthesized or produced from naturally occurring polynucleotides. In addition, the bases in a probe can be joined by a linkage other than a phosphodiester bond, so long as it does not interfere with hybridization. Thus, probes may be peptide nucleic acids in which the constituent bases are joined by peptide bonds rather than phosphodiester linkages (see, for example, Nielsen et al., 1991). Some probes may have leading and/or trailing sequences of noncomplementarity flanking a region of complementarity.

"Hybridization" refers to binding between a nucleic acid probe and a target sequence via complementary base pairing; the resulting complex is referred to as a "hybridization complex". A hybridization complex may be either a complementary complex or a mismatch complex.

"Substantially complementary" means that the sequence of the target is not exactly complementary to the sequence of the probe; however, there is sufficient complementarity that a mismatch complex can form.

"Specific hybridization" refers to the binding, duplexing, or hybridizing of one nucleic acid molecule only to a particular nucleotide sequence under stringent conditions when that sequence is present in a complex mixture (e.g., total cellular) DNA or RNA.

A large number of genes is known to have an effect on body weight of animals, including for instance the genes for the pituitary-specific transcription factor PIT-1; the leptin (LEP) protein; the muscle regulatory factors MyoD1, myogenin, myf-5, and myf-6; cell cycle inhibitor p21 (CDKN1A); retinoblastoma (R,B1); insulin-like growth factors (IGF) 1 and 2; the cyclin-dependent kinase-2 (CDK2); the sarcomeric protein titin (TTN) or connectin; the attenuated form of familial adenomatous polyposis (AFAP); the basic muscle protein myosin; and the TGFβ superfamily of extracellular regulators of cell growth and differentiation which includes several Transforming Growth Factors (TGF's), Bone Morphogenetic Proteins (BMP's), and Growth and Differentiation Factors (GDF's) as well as Mullerian Inhibiting Substance (MIS), inhibin, and myostatin (GDF-8).

It was recently suggested that myostatin plays a role in controlling skeletal muscle growth and differentiation, as well as in regulation of adipose growth in chicken. It was also suggested that SNPs identified in the myostatin gene could be used as a genetic marker for marker assisted selection on chicken growth traits (Zhiliang G, Dahai Z, Ning L, Hui L, Xuemei D, Changxin W. Sci China C Life Sci , 2004 Feb,47(1):25-30).

There is however no suggestion in the prior art that a higher weight at market age of turkey (i.e. body weight at up to approximately 22 weeks) could be obtained by selecting for sequence polymorphisms in the turkey myostatin gene and thus could be useful in marker assisted selection procedures.

In general, good reproductive characteristics such as fertility are negatively correlated with good growth characteristics. For instance double muscled cattle show reduced fertility compared to normal cattle. Thus, breeding for turkeys, in particular male turkeys, having good growth characteristics such as high bodyweight may result in lines of which the hens have poor reproductive characteristics (e.g. having lower egg numbers, reduced egg size, clutch size, fertility, and/or egg hatchability), which will hamper the possibility and increase the cost of propagating the line. The present invention now allows for the production of turkey lines wherein the two traits are uncoupled, or at least wherein the negative impact of genetic improvement of growth on the genetic potential for reproduction is reduced.

The turkey genome is poorly investigated. There are no linkage, cytogenetic or radiation hybrid maps available and very few microsatellite markers. Of all 4046 turkey sequences in the NCBI database of December 2005, 1928 represent expressed sequences (890 ESTs and 1038 mRNAs) the remaining sequences relating to microsatellite markers (~ 250). Nonetheless, the present inventors have discovered that mutations in the myostatin gene correlate with desirable growth characteristics.

The present inventors have identified 23 SNPs in the turkey GDF8 gene. For two SNPs an association study on 1400 animals and several growth and meat traits was performed. A significant association is detected between SBE1 and several growth traits in a cross between two commercial lines that differ enormously in several growth traits. Animals that are homozygous G for SBE1 have approximately 400 grams more body weight at 120 days than animals that are homozygous A for SBE1. In total, this SNP (SBE1) explains 5,25 percent of the total variation in body weight in this cross. The frequencies of the G allele in several commercial lines are in line with the expectations based on the breeding goals that have been set in the last decades.

The present invention provides for a method of selecting a bird of the species *Meleagris gallopavo* that has improved body weight gain characteristics, the body weight gain being the result of improved muscle development, and are preferably exhibited as higher growth rate and/or higher weight at market age. The method is essentially performed on a population of candidate birds and comprises the steps of screening the genome of said candidate birds for the presence of at least one mutation, preferably an SNP, in the myostatin gene associated with improved body weight gain characteristics. The screening step may comprise obtaining a nucleic acid sample, such as for instance a DNA or RNA sample, from each bird of said population of candidate birds, and screening the nucleic acid sample for the presence of at least one SNP in the myostatin gene associated with improved body weight gain characteristics.

Methods for providing a nucleic acid sample of a bird are well known in the art. For instance, a tissue or blood sample may be obtained and DNA may be extracted from the cells comprised therein. Alternatively, nucleic acid may be isolated from eggs and the nucleic acid sample may be screened for the presence of the mutation or SNP. Also, in situ methods may be used, wherein no prior nucleic acid isolation procedures are performed, but wherein the mutation or SNP is directly detected in genome of the for instance an intact cell. Such methods are well within the purview of one of ordinary skill.

Methods for detecting mutations or SNPs in a specified gene are also well known to the skilled person. Mutations or SNPs associated with improved body weight gain characteristics can for instance be identified by comparing the gene sequences of bird lines that have improved body weight gain characteristics with bird lines that have normal or poor body weight gain characteristics. An exemplary method for detecting SNPs associated with improved body weight gain characteristics is described in the Examples below.

It is well within reach of the skilled person to determined whether a mutation is associated with a specific trait. One suitable method, as described in the Examples, combines information on linkage and linkage-disequilibrium. Linkage information refers to recombinations within the marker-genotyped generations and linkage disequilibrium to historical recombinations before genotyping started. Essentially, the analysis consist of three steps; The first step of the analysis is to infer linkage phases of the marker alleles of the genotyped animals such that their paternally and maternally inherited marker haplotypes can be constructed. The second step is the prediction of Identity by descent (IBD) probabilities of pairs of haplotypes at putative QTL positions, thus producing a matrix of IBD probabilities. The IBD probabilities at the QTL position may be predicted using the method of Meuwissen and Goddard, 2001 (Meuwissen THE, Goddard ME (2001) Prediction of identity by descent probabilities from marker-haplotypes. Genet. Sel. Evol. 33:605-634). The third step is the calculation of the likelihood at putative QTL positions using variance component methods. The growth of phenotype individuals was modeled by

$$y = \mu\mathbf{1} + \mathbf{Zh} + \mathbf{u} + \mathbf{e},$$

where $\mu$ is the overall mean, 1 is a vector of ones, Z is an incidence matrix that indicates which haplotypes pertain to which records, h is a vector of random haplotype effects, u is a vector of random polygenic effects (combined effect of background genes), and e is a vector of random sampling errors. The correlation matrices of h and u were $G_p$ and A, respectively, where A is the additive genetic relationship matrix based on the pedigree of the individuals. The variances of the random effects, h, u, and e, were estimated using the ASREML package (Gilmour, A.R., Cullis, B.S., Welham, S.J., Thompson, R. (2000). ASREML Reference Manual. NSW Agriculture, Orange Agric. Inst. Orange, Australia.), with which the likelihood of the above model may also be calculated.

The population of candidate birds may comprise one or more birds and may be a population of birds of a single variety or line. The population preferably consists of birds of different lines, i.e., birds of different varieties, so that the method may essentially be used to uncover a variety that has the requisite growth traits. The method may be performed on birds of an elite flock or on birds of a multiplication stock (i.e. great grand parents, grand parents or parents). Alternatively, the method may be performed on birds of a commercial growing stock. A method of selecting a bird may therefore result in the selection of a bird from a commercial growing stock, having desired weight gain characteristics. Preferably, the method is performed on birds of a turkey nucleus breeding program (i.e. an elite flock). The screening may be performed on birds from any source, including wild birds and birds derived from gene transfer and gene manipulation.

Comparing the myostatin gene sequences of different birds is within the reach of one of ordinary skill in the art. For instance, the myostatin gene may be amplified from the nucleic acid sample using conserved amplification primers (primers directed against conserved regions of the gene sequence). Once such an initial conserved region is successfully amplified and its sequence is obtained, the artisan will understand that additional primers may be designed from which sequencing of the entire gene may commence. Several suitable primers for amplifying myostatin gene sequences are disclosed herein.

Preferably, a method of the invention comprises screening the genome of a candidate bird for the presence of at least one SNP in the myostatin gene associated with improved body weight gain characteristics selected from the group consisting of SNPs numbered 1 to 23 as shown in Table 1.

Table 1. SNPs showing association with improved body weight gain characteristics in the genomic region encoding the GDF8 gene in chicken.

| SNP # | Indication | Location | WT | | Mutant | | Nucl. Position (SEQ ID NO:1) |
|---|---|---|---|---|---|---|---|
| | | | codon | nucl. | codon | nucl. | |
| SNP1 | 791A>T | promoter | - | A | - | T | 791 |

(continued)

| SNP # | Indication | Location | WT | | Mutant | | Nucl. Position (SEQ ID NO:1) |
|---|---|---|---|---|---|---|---|
| | | | codon | nucl. | codon | nucl. | |
| SNP2 | 890C>T | promoter | - | C | - | T | 890 |
| SNP3 | 1467C>T | exon1 | TGC | C | TGT | T | 1467 |
| SNP4 | 1823G>C | intron1 | - | G | - | C | 1823 |
| SNP5 | 1917T>G | intron1 | - | T | - | G | 1917 |
| SNP6 | 2139>indel | intron1 | - | - | - | CA | 2139 |
| SNP7 | 2278C>G | intron1 | - | C | - | G | 2278 |
| SNP8 | 2299A>G | intron1 | - | A | - | G | 2299 |
| SNP9 | 3314G>A | intron2 | - | G | - | A | 3314 |
| SNP10 | 3537C>T | intron2 | - | C | - | T | 3537 |
| SNP11 | 3538A>G | intron2 | - | A | - | G | 3538 |
| SNP12 | 3545T>C | intron2 | - | T | - | C | 3545 |
| SNP13 | 3546T>C | intron2 | - | T | - | C | 3546 |
| SNP14 | 3549C>T | intron2 | - | C | - | T | 3549 |
| SNP15 | 3553G>T | intron2 | - | G | - | T | 3553 |
| SNP16 | 3669T>A | intron2 | - | T | - | A | 3669 |
| SNP17 | 3766A>G | intron2 | - | A | - | G | 3766 |
| SNP18 | 4419G>A | 3'UTR | - | G | - | A | 4419 |
| SNP19 | 4692C>T | 3'UTR | - | C | - | T | 4692 |
| SNP20 | 4774C>T | 3'UTR | - | C | - | T | 4774 |
| SNP21 | 4830C>T | 3'UTR | - | C | - | T | 4830 |
| SNP22 | 5300A>G | 3'UTR | - | A | - | G | 5300 |
| SNP23 | 5406G>T | 3'UTR | - | G | - | T | 5406 |

The present invention also relates to a method for producing a bird of the species *Meleagris gallopavo* comprising selecting a bird of the species *Meleagris gallopavo* according to a method of the invention as described above, crossing the thus selected bird with a bird of a desirable turkey line and hatching the fertilized egg to produce the bird.

In general, in case the bird is a bird of an elite flock or multiplication stock, the bird selected by a method of the invention will have many other (commercially) desirable characteristics such as for instance moderate fat deposition, resistance to disease, good body conformation and excellent feed efficiency that are the result of previous selective breeding efforts. However, the selected bird does not necessarily have to be a specimen from an elite flock. The selected bird may for instance also merely be a source of a desired myostatin gene that is to be introduced into a desirable elite flock or multiplication stock background by crossing and progressive inbreeding. The skilled person is well acquainted with such methods. Preferably, however, the desirable turkey line from which the bird is selected is an elite flock or a multiplication stock.

Crossing of birds may be performed by natural mating but preferably occurs by artificial insemination. Conditions for hatching of fertilized eggs are known in the art. A method for producing a bird of the species *Meleagris gallopavo* according to the present invention always comprises as an essential element the step of screening the nucleic acid from birds for the presence of the mutations in the myostatin gene as described above, wherein said mutations are preferably SNPs, and most preferably SNPs selected from the group consisting of SNPs numbered 1 to 23 as shown in Table 1 above.

The present invention also relates to a method wherein essentially the number and frequency of mutations in the myostatin gene, associated with improved body weight gain characteristics, is increased in a turkey line. In this way a turkey line having improved body weight gain characteristics, resulting from improved muscle deposition, may be produced. Again, the body weight gain characteristics are preferably higher growth rate and/or higher weight at market age. The mutations for which the frequency is increased are preferably SNPs selected from the group consisting of SNPs numbered 1 to 23 as shown in Table 1 above.

Turkey lines, including elite flocks, are not necessarily homogeneous for certain genetic traits. For instance, certain mutations in the myostatin gene may be present in some birds of the flock, but may be absent in others. When using such birds for breeding, the chances are that the offspring birds (great grandparents, grandparents or parents) do not receive the desired gene sequence from their parent. Therefore a method for producing a turkey line having improved body weight gain characteristics according to the invention is essentially a method of increasing the number of desired mutations in the genome of individual birds, thereby improving the genetic constitution of the individual bird, and/or increasing the percentage of birds having desired mutations, thereby improving the uniformity of the flock or line for the genetic constitution. Preferably both the referred number as well as the referred frequency are increased.

A method for producing a turkey line having improved body weight gain characteristics according to the invention as described above may very suitably comprise a method for producing a bird of the species *Meleagris gallopavo* having improved body weight gain characteristics according to the invention, which method includes the steps of selection and crossing as described. Most preferably, the improved body weight gain characteristics are not associated with reduced egg production in female birds. Thus, male lines there is a desire to increase the number of mutations in the myostatin gene as described herein and/or the frequency of the mutations. In female lines, the increase of number and frequency of mutations may or may not be desirable depending on the breeding goal for that line. Most importantly, the mutations that are allowed in female lines must not strongly negatively influence the reproductive characteristics of that line.

A bird of the species *Meleagris gallopavo* having improved body weight gain characteristics, wherein said body weight gain is obtained by improved muscle deposition, and wherein said bird has at least one, more preferably at least two, still more preferably at least 6 single nucleotide polymorphisms (SNP) in the myostatin gene associated with improved body weight gain characteristics as described herein. Although such birds may exist in nature, their importance in turkey breeding has hitherto not been recognized. Preferably the mutations are SNPs as described herein and more preferably SNPs selected from the group consisting of SNPs numbered 1 to 23 as shown in Table 1. The body weight gain characteristics are preferably higher growth rate and/or higher weight at market age, and the SNPs are preferably mutations in an intron sequence. The bird may be obtained by using the selection methods of the present invention or by using the production methods as described above.

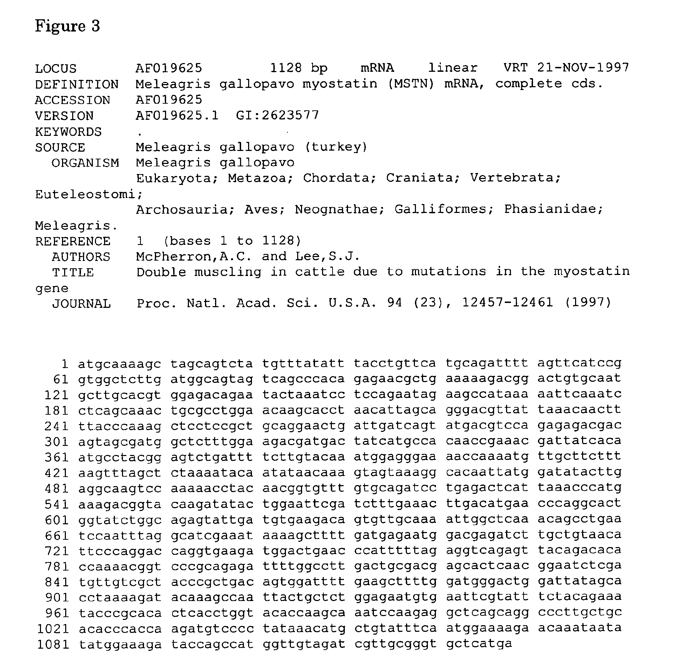

The present invention also relates to a turkey line obtainable by a method according to any one of claims. A characteristic of such a line may for instance be that the number of mutations in the myostatin gene is increased by selective breeding and screening as described herein. In addition or alternatively, the frequency of the mutations in the myostatin gene may be increased. Preferably, not only does a single bird have more mutations in the myostatin gene relative to reference sequence of GenBank accession AF019625 (SEQ ID No. 2 as provided in Figure 3), but also the percentage of birds within that line that have the increased number of mutations is increased.

A turkey line of the invention is preferably an elite flock, a multiplication stock or a flock of commercials.

A turkey line may also be obtained by other methods. The features of such a turkey line are a number of mutations in the myostatin gene of at least 1, preferably at least 2, more preferably at least 3, 4 or 5, even more preferably 6, 7 or 8, mutations in the myostatin gene associated with improved body weight gain characteristics. Most preferably, the mutations are SNPs of Table 1. Alternatively, or in addition to the elevated mutation number a turkey line of the present invention may exhibit a frequency of the mutated gene, expressed as the percentage of birds of said flock having the desired mutation, of at least 80%, more preferably at least 90%, even more preferably at least 95%, most preferably at least 98 or 99%.

The methods of the present invention may be used in a turkey nucleus breeding program in addition to traditional genetic improvement programs for turkeys, but as indicated, the methods of the present invention may also be used outside nucleus breeding programs to identify turkeys with superior weight gain and muscle deposition characteristics.

The methods of the present invention may be used to identify individual birds that are expected to give progeny with superior characteristics for commercial meat growing in turkeys or that are expected to show superior meat growing characteristics in their own right.

A specific attribute of the method is that it can be applied at a very young age of the animals: as the method is based on the analysis of nucleic acod samples of individual birds it may be applied immediately after hatching of the birds, or, indeed, at the embryonic stage.

Other aspects foreseen in the present invention are methods for screening turkeys for presence or absence of mutations in the myostatin gene. Such screening methods may involve the use of nucleic acid detection techniques such as PCR (Mullis 1987, U.S. Pat. No. 4,683,195, 4,683,202, and 4,800,159) - optionally in a nested, multiplex or a-symmetrical format - or by using the ligase chain reaction (Barany 1991, Proc. Natl. Acad. Sci. USA 88:189-193; EP Application No., 320,308), self sustained sequence replication (3SR) (Guatelli et al., 1990, Proc. Natl. Acad. Sci. USA 87:1874-1878), strand displacement amplification (SDA)" (U.S. Pat. Nos. 5,270,184, and 5,455,166), transcriptional amplification system (Kwoh et al., Proc. Natl. Acad. Sci. USA 86:1173-1177), Q-Beta Replicase (Lizardi et al., 1988, Bio/Technology 6:1197), Rolling Circle Amplication (RCA) or any other method for amplifying nuclei acids. In an alternative method, RNA may be used directly or be amplified prior to use by RNA amplification methods such as Nucleic Acid Sequence-Based Amplification (NASBA) (L. Malek et al., 1994, Meth. Molec. Biol. 28, Ch. 36, Isaac PG, ed., Humana Press, Inc., Totowa, N.J.) or Transcription Mediated Amplification (TMA). Also nuclei acid arrays may be used. Methods for obtaining genetic information by using nuclei acid arrays are well known in the art (see i.a. Chee et al., 1996, Science 274(5287):610-614). In a screening method according to the present invention, the application of a DNA microarray may be preferred. Such arrays of oligonucleotides comprise for instance DNA sequences that are specific for certain genetic markers.

Most preferred for screening purpose is the use of PCR. The PCR method is well described in handbooks and known to the skilled person.

PCR is essentially a template-dependent extension of the oligonucleotide primer(s) catalyzed by a polymerizing agent in the presence of adequate amounts of the four deoxyribonucleotide triphosphates (dATP, dGTP, dCTP and dTTP, i.e. dNTPs) or analogues, in a reaction medium which is comprised of the appropriate salts, metal cations, and pH buffering system. Suitable polymerizing agents are enzymes known to catalyze primer- and template-dependent DNA synthesis. Known DNA polymerases include, for example, E. coli DNA polymerase I or its Klenow fragment, T4 DNA polymerase, and Taq DNA polymerase. The reaction conditions for catalyzing DNA synthesis with these DNA polymerases are known in the art.

The term "primer" as used herein refers to an oligonucleotide which is capable of annealing to the amplification target allowing a DNA polymerase to attach, thereby serving as a point of initiation of DNA synthesis when placed under conditions in which synthesis of primer extension product is induced, i.e., in the presence of nucleotides and an agent for polymerization such as DNA polymerase and at a suitable temperature and pH. The (amplification) primer is preferably single stranded for maximum efficiency in amplification. Preferably, the primer is an oligodeoxyribonucleotide. The primer must be sufficiently long to prime the synthesis of extension products in the presence of the agent for polymerization. The exact lengths of the primers will depend on many factors, including temperature and composition (A/T en G/C content) of primer. A pair of bi-directional primers consists of one forward and one reverse primer as commonly used in the art of DNA amplification such as in PCR amplification.

It will be understood that "primer", as used herein, may refer to more than one primer, particularly in the case where there is some ambiguity in the information regarding the terminal sequence(s) of the target region to be amplified. Hence, a "primer" includes a collection of primer oligonucleotides containing sequences representing the possible variations in the sequence or includes nucleotides which allow a typical base pairing. Very suitable primers for detection of SNPs are single base extension primers (SBEs) which are well known in the field of SNP detection.

The oligonucleotide primers may be prepared by any suitable method. Methods for preparing oligonucleotides of specific sequence are known in the art, and include, for example, cloning and restriction of appropriate sequences, and direct chemical synthesis. Chemical synthesis methods may include, for example, the phospho di- or tri-ester method, the diethylphosphoramidate method and the solid support method disclosed in e.g. U.S. Pat. No. 4,458,066. The primers may be labeled, if desired, by incorporating means detectable by for instance spectroscopic, fluorescence, photochemical, biochemical, immunochemical, or chemical means.

The products of the primer- and template-dependent DNA synthesis are duplex molecules consisting of the template strands and the primer extension strands, which include the target sequence. These products, in turn, serve as template for another round of replication. In the second round of replication, the primer extension strand of the first cycle is annealed with its complementary primer; synthesis yields a "short" product which is bound on both the 5'- and the 3'-ends by primer sequences or their complements. Repeated cycles of denaturation, primer annealing, and extension result in the exponential accumulation of the target region defined by the primers. Sufficient cycles are run to achieve the desired amount of polynucleotide containing the target region of nucleic acid. The desired amount may vary, and is determined by the function which the product polynucleotide is to serve.

After amplification by PCR, the target polynucleotides may be detected by hybridization with a probe polynucleotide which forms a stable hybrid with that of the target sequence under stringent to moderately stringent hybridization and wash conditions. If it is expected that the probes will be essentially completely complementary (i.e., about 99% or greater) to the target sequence, stringent conditions will be used. If some mismatching is expected, for example if variant strains are expected with the result that the probe will not be completely complementary, the stringency of hybridization may be lessened. However, conditions are chosen which rule out nonspecific/adventitious binding. Conditions which affect hybridization, and which select against nonspecific binding are known in the art, and are described in, for example, Sambrook, J., E.F. Fritsch, and T. Maniatis. Molecular Cloning: a Laboratory Manual (3rd. edition). Cold Spring Harbor Laboratory Press, Plainview, N.Y., 2000. Generally, lower salt concentration and higher temperature increase the stringency of binding. For example, it is usually considered that stringent conditions are incubations in solutions which contain approximately 0.1xSSC, 0.1% SDS, at about 65°C incubation/wash temperature, and moderately stringent conditions are incubations in solutions which contain approximately 1-2xSSC, 0.1% SDS and about 50°-65°C incubation/wash temperature. Low stringency conditions are 2xSSC and about 30°-50°C.

The terms "stringency" or "stringent hybridization conditions" refer to hybridization conditions that affect the stability of hybrids, e.g., temperature, salt concentration, pH, formamide concentration and the like. These conditions are empirically optimised to maximize specific binding and minimize non-specific binding of primer or probe to its target nucleic acid sequence. The terms as used include reference to conditions under which a probe or primer will hybridize to its target sequence, to a detectably greater degree than other sequences (e.g. at least 2-fold over background). Stringent conditions are sequence dependent and will be different in different circumstances. A variety of factors may significantly affect the stringency of hybridization, including, among others, base composition, size of the complementary strands, the presence of organic solvents and the extent of base mismatching; the combination of parameters is more important than the absolute measure of any one. For a discussion of general factors influencing hybridization, see for example, WO 93/02216 and Watson et al., Recombinant DNA, 2.sup.nd Edition, Scientific American Books, NY 1992, each of which is incorporated herein by reference in its entirety. An extensive guide to hybridization of nucleic acids is found in Ausubel et al., Current Protocols in Molecular Biology,Greene Publishing Associates, Inc. and John Wiley and Sons, Inc. (supplemented through 1998), which is also incorporated herein by reference in its entirety. Generally, stringent conditions are selected to be about 5°C lower than the thermal melting point (Tm) for the specific sequence at a defined ionic strength and pH. The Tm is the temperature (under defined ionic strength and pH) at which 50% of a complementary target sequence hybridises to a perfectly matched probe or primer. Typically, stringent conditions will be those in which the salt concentration is less than about 1.0 M Na+ ion, typically about 0.01 to 1.0 M Na+ ion concentration (or other salts) at pH 7.0 to 8.3 and the temperature is at least about 30°C for short probes or primers (e.g. 10 to 50 nucleotides) and at least about 60°C for long probes or primers (e.g. greater than 50 nucleotides). Stringent conditions may also be achieved with the addition of destabilizing agents such as formamide. Exemplary low stringent conditions or "conditions of reduced stringency" include hybridization with a buffer solution of 30% formamide, 1 M NaCl, 1% SDS at 37°C and a wash in 2x SSC at 40°C. Exemplary high stringency conditions include hybridization in 50% formamide, 1 M NaCl, 1% SDS at 37°C, and a wash in 0.1x SSC at 60°C. Hybridization procedures are well known in the art and are described in e.g. Ausubel, et al. eds (1995) Current Protocols in Molecular Biology*,* and Sambrook J, and Russell DW (2001). Molecular Cloning: A Laboratory Manual. New York, NY, USA., Cold Spring Harbor Laboratory Press.

The screening methods may be performed on a nucleic acid sample from a single individual bird, or on pooled genomic DNA. The term "pooled genomic DNA" refers to a physical mixture of DNA samples obtained by combining the isolated DNA resulting from DNA extraction procedures on different samples or on different strains.

The methods of the invention may be used as part of or as en extension of current genetic selection program in pure breeding lines of turkeys.

The methods of the invention may be used during multiplication stages of turkey breeding stock which is being multiplied and/or hybridized to produce turkeys for grow out and meat production.

The methods of the invention may be used for testing of animals in multiplication or final grow out stages in order to create more uniform groups of animals for supply to the next production stage, where the next production stage may be hatching egg production, grow out of turkeys to market age, or processing or further processing of killed animals and their carcasses.

The screening methods themselves for as far as they relate to detection of the SNPs of Table 1 are also an aspect of the present invention

Although in most instances the number of mutations in the myostatin gene are beneficially increased in individual birds, the present application also envisages the selection and production of birds having decreased frequency of specific alleles of myostatin. This may for instance be the case where certain alleles (e.g. certain SNPs) are undesirable in female lines. In this way, the characteristics of the resulting population can be changed.

## EXAMPLES

### Materials and Methods

#### Animals

An F2 cross between two commercial Hybrid lines was made. One line is a male line selected for weight for several generations. The other line is a female line that is highly productive but slow growing. Eighteen F1 males were mated with 17 F1 females to produce approximately 1400 F2 offspring. For all F1 and F2 animals blood samples were taken. Genomic DNA was isolated from these blood samples following standard protocols.

#### Phenotypes

Phenotypes were collected for F1 and F2 animals. In total, 32 different traits were measured on different subsets of animals. Several weight traits (e.g. body weight at different ages, breast and shank length) and meat quality traits (e.g. pH, temperature, drip, colour) were measured. These traits were all measured using standard protocols.

Amplification and sequencing of the turkey GDF8 gene

Primers for amplifying parts of the turkey GDF8 gene were designed based on the published turkey mRNA sequence AF019625 (SEQ ID No. 2) and the chicken mRNA sequence AF346599 from the NCBI Entrez Nucleotide database (http://www.ncbi.nlm.nih.gov/entrez/). These amplified parts of the turkey GDF8 gene were used for detection of SNPs between several birds. Primers were designed in such a way that both exonic and intronic regions could be amplified (Table 2).

**Table 2** Primers designed for GDF8 exon and intron amplification.

| Part of gene amplified | Name | Primer sequences | Identity |
|---|---|---|---|
| Promotor | PCR1 | F: gcttaatgcacgtgaactgttg | SEQ ID No. 3 |
| | | R: ctaaacagatccgggacagc | SEQ ID No. 4 |
| Exon 1 | PCR2 | F: gggctttaacctctgacagc | SEQ ID No. 5 |
| | | R: gggagagcctgagaaggagt | SEQ ID No. 6 |
| Intron 1 | PCR3 | F: tacccaaagctcctccactg | SEQ ID No. 7 |
| | | R: agcagatgtttagcactaagaagat | SEQ ID No. 8 |
| Exon 2 | PCR4 | F: ttgcatccactctgttaccaa | SEQ ID No. 9 |
| | | R: cattctcgaagcaatatttcca | SEQ ID No. 10 |
| Intron 2 | PCR5 | F: gaatccaatttaggcatcgaa | SEQ ID No. 11 |
| | | R: cagaatgatcaattgtgtagcag | SEQ ID No. 12 |
| Exon 3 | PCR6 | F: tcctcaaattacatacgcctacc | SEQ ID No. 13 |
| | | R: gttggcaatgcctagcgta | SEQ ID No. 14 |
| 3' UTR | PCR7 | F: tacgctaggcattgccaac | SEQ ID No. 15 |
| | | R: ctcagcctgcttacgtagca | SEQ ID No. 16 |
| 3' UTR | PCR8 | F: tgctacgtaagcaggctgag | SEQ ID No. 17 |
| | | R: gagaaaacctgggtttgtttatt | SEQ ID No. 18 |

The PCR reactions were carried out in 24 µl volumes. Reactions contained 6 µl DNA (10 ng/µl), 12 µl AB-gene Master Mix, 2 µl primermix (both primers in a conc. of 30 ng/µl) and 4 µl MQ. Reactions were performed in a PCR machine with heated lid, e.g. PTC100 thermocycler (MJ Research, Watertown, MA, USA) with a 5 min initial denaturation at 95°C followed by 36 cycles of 30 s at 95°C, 45 s at annealing temperature and 90 s at 72°C, and a final extension for 10 min at 72°C. The annealing temperatures were optimized for each primer pair and varied between 50°C and 62°C.

After PCR the excess of primers was removed by running the samples over MANU030 PCR columns in 96-well format (Millipore, USA). From each sample, 2 µl was checked on agarose gel to estimate the DNA concentration. Sequencing reactions were performed with either the forward or the reverse amplification primer. Cycle sequencing reactions contained 100-400 ng of purified PCR product, 1 µl of Big Dye Terminator Rtmix version 2.0 (PE, Foster City, CA, USA), 3 µl of Half Big Dye Buffer (Genetix, New Milton, UK) and 0.8 pmol of either primer in a final volume of 10 µl. Excess dye terminator was removed by purification of the samples on Montage SEQ96 columns in 96-well format (Millipore, USA). After purification, the DNA is held in 15 µl Injection Solution (Millipore, Bedford, MA, USA). 2 µl is mixed with 10 µl formamide and denatured for 2 min. at 95°C before analysis on an ABI 3100 automated sequencer (ABI, Foster City, CA, USA).

SNP detection

The SNP discovery panel consisted of eight F1 animals. (animal ID 4143, 4146, 4104, 4156, 4073, 4081, 4094, 4206). These animals represent the parents that produced the largest part of the F2 animals.

Trace files were analyzed using the Pregap4 program of the Staden software package (Staden, R. 1996. The Staden Sequence Analysis Package. Molecular Biotechnology 5, 233-241; Bonfield JK and Staden R. (1996). Experiment files and their application during large-scale sequencing projects. DNA Sequence 6, 109-117.; http://www.mrc-lmb.cam.ac.uk/pubseq). Using Pregap4, all chromatograms were analyzed: bases were called with Phred (Ewing B, Hillier L, Wendl MC, Green P. 1998. Base-calling of automated sequencer traces using Phred. I. Accuracy assessment. Genome Res. 8(3):175-185) and low quality regions were masked. Next, all sequences passing Pregap4 were entered in a Gap4 database. In the Gap4 program (Bonfield, J.K., K. Smith, and R. Staden. 1995. A new DNA sequence assembly program. Nucleic Acids Res. 24: 4992-4999) a normal shotgun assembly was performed with a minimal initial match of 20 bp, a maximum of 25 pads per read and a maximum of 5 % mismatches. If necessary, the assembly was finished manually using the Gap4 *Join contig* interface. Positions at which a disagreement occurred were highlighted in the Gap4 contig editor window using the *Highlight disagreements* option. All nucleotide positions at which a disagreement occurred were tagged as a putative SNP position.

Design of SBE primers

Single-base extension (SBE) primers were designed to have a specific 3'end 18-25 bp in length. A non-specific 5'tail was used to create primers 25-120 bp in length, with 5 bp intervals. Two sets of SBE primers were designed; one initial set containing only 2 SNP detected in a preliminary test (Table 3) and one set with 8 SNPs detected in additional PCR fragments amplified based on the chicken and turkey sequences (Table 4).

**Table 3.** First SBE set designed for typing two SNPs in the turkey GDF8 gene

| Position in gene | Name | Nt* | SBE primer | SEQ ID No |
|---|---|---|---|---|
| Intron 2 | SBE1 | 163 | T(38) GAGGATTTAGTATTCTGTCTCCACGT | 19 |
| 3' UTR | SBE2 | 459 | T(48) CATCGTGAGATCCTCCACCTC | 20 |

* position in PCR fragment (The product size is the length of the primer plus 1 base)

**Table 4.** Second SBE set designed for typing eight SNPs in the turkey GDF8 gene

| Position in gene | Name | Nt* | SBE primer | | SEQ ID No. |
|---|---|---|---|---|---|
| Promoter | SBE3 | 69 | tttttttttttttt t | TTCCTAATGTTTGCAGTGTGTTTATTATGTACTT | 21 |
| Promoter | SBE4 | 168 | T(23) | GACAGAATCCTAAAGCAACTCTAAAGAAAGC | 22 |
| Intron 1 | SBE5 | 586 | T(36) | TGCGAGCCTTTGTTTTACTGTTCATTT | 23 |
| Intron 1 | SBE6 | 492 | T(42) | CCTCCCTCCGCAGACTC | 24 |
| Intron 2 | SBE7 | 269 | | ATTAATGACAGTGAGAATTATCTATGTTTATGAATCCTAAATTG | 25 |
| Intron 2 | SBE8 | 137 | T(29) | GTGGTTAAACTGGTATGCTTTAGATTTATTATGTTCATC | 26 |
| 3' UTR | SBE9 | 263 | ttttttttt | AGGACAGAAGGAGCAGGCTA | 27 |
| 3 UTR | SBE10 | 207 | tttttttt | GTGGTATTGTGTAGTGGTGTATATGTTACC | 28 |

* position in PCR fragment. (The product size is the length of the primer plus 1 base)

Typing of SNPs on complete F2 population

After identification of SNPs from the 8 individuals in the SNP discovery panel, the entire population of F1 parents and their 1400 offspring were genotyped for these SNPs by single-base extension (SBE). SBE genotyping was performed using the multiplex SNaPshot kit (ABI, Foster City, CA, USA) following the protocol provided by the manufacturer. SNaPshot products were analyzed on an ABI3100 automated sequencer. Resulting data were analyzed using the GeneMapper software package (v3.0 ; ABI) and checked by two independent persons.

Association studies

SNPs were genotyped on the entire population of birds from cross between commercial pure lines differing in the selection for growth traits were analyzed using the LDLA (Linkage Disequilibrium / Linkage Analysis) program by Meuwissen et al. 2002 (Meuwissen THE, Karlsen A, Lien S, Olsaker I, and Goddard ME (2002) Fine Mapping of a Quantitative Trait Locus for Twinning Rate Using Combined Linkage and Linkage Disequilibrium Mapping. Genetics 161:373-379).

In this program, in the first step linkage phases of the marker alleles are inferred to construct their paternally and maternally inherited marker haplotypes. In the second step, IBD probabilities of pairs of haplotypes at putative mutation or QTL positions are predicted. The third step involves the calculation of the LogLikelihood at putative mutation or QTL positions using variance component methods. After that twice the reduction in LogLikelihood was tested, resulting from dropping t random terms as a Chisquare statistic. The 5% significance threshold for this test statistic is 3.9.

Frequencies of SBEs in pooled samples of several commercial lines

To estimate the frequencies of the SNPs detected in this study, all eight SBEs were typed on pooled DNA samples of several commercial lines. Between 50 and 75 individuals per commercial line were pooled. Equal amounts of blood of all these individuals were pooled and mixed. DNA extraction of this pooled samples was performed using standard protocols.

Results

Amplification of the turkey GDF8 gene

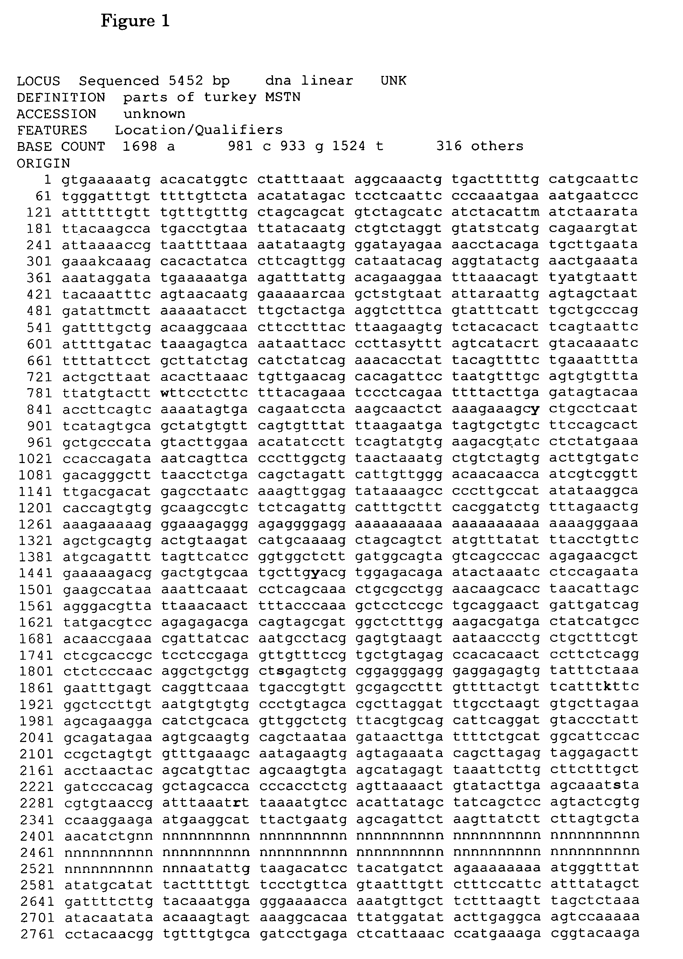

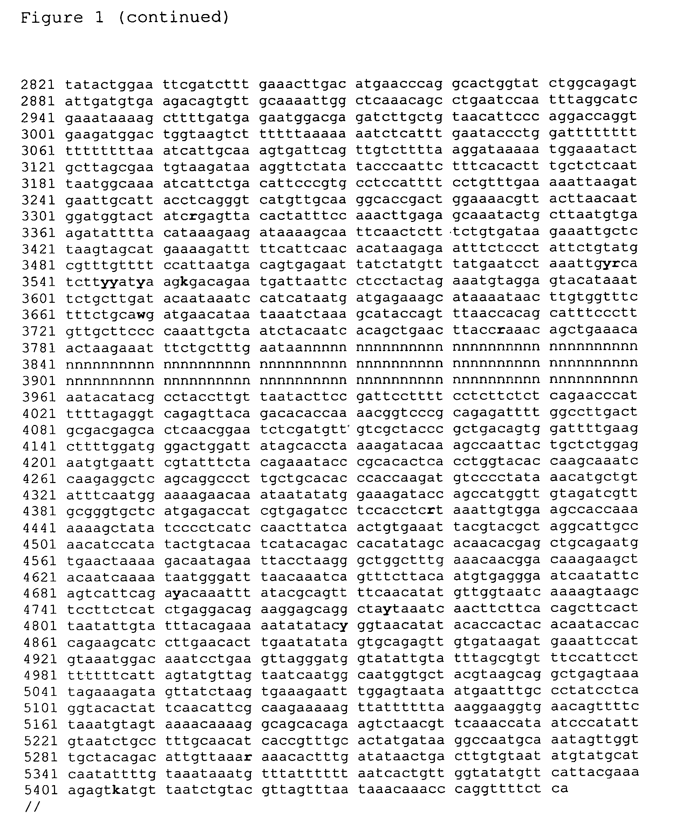

Sequences for the turkey GDF8 gene corresponding to exons 1,2, and 3, intron 1 and 2, parts of the promoter region and the 3' untranslated region were generated (Figure 1). The exonic sequences showed very high percentage identity with turkey mRNA sequence AF019625 (SEQ ID No. 2) and a slightly lower percentage identity with the chicken mRNA sequence AF346599.

Sequencing and SNP detection in the turkey GDF8 gene

Sequencing of the PCR fragments covering a large part of the turkey GDF8 gene on the sequencing test panel of 8 animals from a commercial cross between the commercial pure lines differing in the selection for growth traits resulted in a 23 SNPs between these animals (Table 5).

SNPs were detected mainly in intronic sequences (14 SNPs), promotor (2 SNPs) and 3' UTR (6 SNPs) sequences. Only SNP was detected in an exon, a C to T transition in exon 1. Since this SNPs is a synonymous mutation, it did not result in a amino acid change.

As can be seen from Table 5, SNPs can be grouped in several haplotype blocks. Based on the 8 animals in the SNP discovery panel, none of the detected SNPs was completely associated with the phenotype. Based on literature and since growth traits are linear traits, it is not expected that one SNP will explain all the variance for growth in a population.

**Table 5.** SNPs detected in all the PCR fragments. Genotypes are indicates for each animal in the SNP discovery panel. If a SNP is typed on the complete F2 population, its SBE number is indicated

| PCR1 | | Nt* | 4143 | 4146 | 4104 | 4156 | 4073 | 4081 | 4094 | 4206 |
|---|---|---|---|---|---|---|---|---|---|---|
| SNP1 | SBE3 | 69 | A/A | A/T | A/T | T/T | A/T | A/T | A/T | A/T |
| SNP2 | SBE4 | 168 | T/T | C/T | C/T | C/T | C/T | C/T | C/T | C/T |

(continued)

| PCR2 | | Nt* | 4143 | 4146 | 4104 | 4156 | 4073 | 4081 | 4094 | 4206 |
|---|---|---|---|---|---|---|---|---|---|---|
| SNP3 | SBE1 | 163 | T/T | C/T | C/T | C/C | C/T | C/T | C/T | C/T |

| PCR3 | | Nt* | 4143 | 4146 | 4104 | 4156 | 4073 | 4081 | 4094 | 4206 |
|---|---|---|---|---|---|---|---|---|---|---|
| SNP4 | SBE5 | 586 | G/G | G/G | G/G | C/G | G/G | G/G | G/G | G/G |
| SNP5 | SBE6 | 492 | T/T | T/T | T/T | G/T | T/T | T/T | T/T | T/T |
| SNP6 | | 270 | - | - | - | indel | - | - | - | - |
| SNP7 | | 131 | C/C | C/G | C/G | - | C/G | C/G | C/G | C/G |
| SNP8 | | 110 | G/G | A/G | A/G | - | A/G | A/G | A/G | A/G |

| PCR4 | | Nt* | 4143 | 4146 | 4104 | 4156 | 4073 | 4081 | 4094 | 4206 |
|---|---|---|---|---|---|---|---|---|---|---|
| No | SNPs | detected | | | | | | | | |

| PCR5 | | Nt* | 4143 | 4146 | 4104 | 4156 | 4073 | 4081 | 4094 | 4206 |
|---|---|---|---|---|---|---|---|---|---|---|
| SNP9 | | 492 | G/G | G/G | G/G | A/G | G/G | G/G | G/G | G/G |
| SNP10 | SBE7 | 269 | T/T | C/T | C/T | C/T | C/T | C/T | C/T | C/T |
| SNP11 | | 268 | G/G | A/G | A/G | A/G | A/G | A/G | A/G | A/G |
| SNP12 | | 261 | T/T | T/T | T/T | C/T | T/T | T/T | T/T | T/T |
| SNP13 | | 260 | T/T | T/T | T/T | C/T | T/T | T/T | T/T | T/T |
| SNP14 | | 257 | C/C | C/T | C/T | T/T | C/T | C/T | C/T | C/T |
| SNP15 | | 253 | G/G | G/T | G/T | T/T | G/T | G/T | G/T | G/T |
| SNP16 | SBE8 | 137 | T/T | T/T | T/T | A/T | T/T | T/T | T/T | T/T |
| SNP17 | | 40 | A/A | A/A | A/A | A/G | A/A | A/A | A/A | A/A |

| PCR6 | | Nt* | 4143 | 4146 | 4104 | 4156 | 4073 | 4081 | 4094 | 4206 |
|---|---|---|---|---|---|---|---|---|---|---|
| SNP18 | SBE2 | 459 | G/G | G/G | G/G | A/G | G/G | G/G | G/G | G/G |

| PCR7 | | Nt* | 4143 | 4146 | 4104 | 4156 | 4073 | 4081 | 4094 | 4206 |
|---|---|---|---|---|---|---|---|---|---|---|
| SNP19 | | 345 | T/T | C/T | C/T | C/C | C/T | C/T | C/T | C/T |
| SNP20 | SBE9 | 263 | C/C | C/T | C/T | T/T | C/T | C/T | C/T | C/T |
| SNP21 | SBE10 | 207 | C/C | C/C | C/C | C/T | C/C | C/C | C/C | C/C |

| PCR8 | | Nt* | 4143 | 4146 | 4104 | 4156 | 4073 | 4081 | 4094 | 4206 |
|---|---|---|---|---|---|---|---|---|---|---|
| SNP22 | | 284 | A/A | A/G | A/G | G/G | A/G | A/G | A/G | A/G |
| SNP23 | | 390 | T/T | G/T | G/T | G/G | G/T | G/T | G/T | G/T |
| *position in PCR fragment | | | | | | | | | | |

Typing of SNPs on complete F2 population

Two SNPs (SBE1 and SBE2) were typed on the complete population. The number of available phenotypes differs per trait. The test statistics resulting from the LDLA analysis are given in Table 6 for body weight at 17, 40, 60, 80 and 120 days (bw17, bw40, bw60, bw80, and bw120). It can be seen from this table that significant association was detected between the SBE1 and Body weight at several ages. The 5% significance threshold for the test statistic is 3.9.

Table 6. LDLA analysis of SBE1 in turkey GDF8 sequence

| Trait | Test statistic | P value | # of animals |
|---|---|---|---|
| Bw 17 | 4.8 | <0.025 | 841 |
| Bw40 | 9.4 | <0.005 | 826 |
| Bw60 | 10.4 | <0.005 | 819 |
| Bw80 | 7.2 | <0.010 | 791 |
| Bw 120 | 9.0 | <0.005 | 740 |

The effect on body weight at 120 days is given in Table 7. Animals that are homozygous G for SBE1 have approximately 400 grams more body weight at 120 days than animals that are homozygous A for SBE1. In total, this SNP (SBE1) explains 5.25 percent of the total variation in body weight.

Table 7. Effects of SBE1

| Genotype | Effect in grams |
|---|---|
| AA | -170 |
| AG | 0 |
| GG | +220 |

Frequencies of SBEs in pooled samples of several commercial lines

To estimate the frequencies of the SNPs detected in this study, all eight SBEs were typed on pooled DNA samples of several commercial lines. Both males and female lines were included. Based on knowledge of breeding goals in the last decades, it is expected that male lines will have high frequencies of genes or SNPs that are associated with growth and meat. Female lines are expected to have much lower frequencies of SNPs associated with growth and meat.

In Table 8 the frequencies for the eight SBEs are given for several commercial lines. One of the two alleles per SNP is represented. The frequency of the other allele is 100% minus the frequency of the represented allele. It can be seen that the G allele of SBE1 has in all lines, both from Hybrid and lines from competitors, clearly a much higher frequency than the A allele.

**Table 8.** Frequencies of the SBEs in different commercial lines.

| Lines | type line* | SBE1 | SBE3 | SBE4 | SBE5 | SBE6 | SBE7 | SBE8 | SBE9 | SBE10 |
|---|---|---|---|---|---|---|---|---|---|---|
| Allele | | G | T | C | T | T | C | T | T | T |
| Hybrid_1 | F | 9,11 | 22,73 | 17,79 | 100,00 | 100,00 | 9,05 | 100,00 | 8,07 | 100,00 |
| Hybrid_2 | F | 0,00 | 0,00 | 0,00 | 100,00 | 100,00 | 0,00 | 69,52 | 34,77 | 100,00 |
| Hybrid_3 | F | 36,37 | 38,28 | 25,87 | 85,73 | 85,62 | 23,28 | 86,23 | 38,05 | 83,73 |
| Hybrid_4 | F | 35,17 | 35,00 | 9,54 | 91,39 | 91,45 | 26,03 | 88,91 | 38,12 | 90,09 |
| Hybrid_5 | F | 43,80 | 35,04 | 0,00 | 100,00 | 58,68 | 7,14 | 65,25 | 47,29 | 100,00 |
| Hybrid_6 | M | 85,13 | 87,07 | 87,80 | 100,00 | 100,00 | 80,36 | 100,00 | 84,61 | 100,00 |
| Hybrid_7 | M | 84,92 | 86,35 | 87,77 | 100,00 | 100,00 | 71,65 | 83,76 | 85,80 | 100,00 |
| Hybrid_8 | F | 47,09 | 34,84 | 3,27 | 100,00 | 52,66 | 0,00 | 50,69 | 47,99 | 100,00 |
| Hybrid_9 | M | 100,00 | 100,00 | 98,04 | 100,00 | 100,00 | 100,00 | 100,00 | 100,00 | 100,00 |
| Comp1_1 | F | 73,42 | 71,60 | 12,60 | 82,50 | 71,66 | 54,15 | 81,40 | 73,65 | 78,72 |
| Comp1_2 | M | 88,06 | 88,22 | 92,48 | 100,00 | 100,00 | 84,20 | 90,99 | 86,21 | 100,00 |
| Comp1_3 | F | 4,62 | 0,00 | 0,00 | 100,00 | 100,00 | 0,00 | 92,65 | 0,00 | 100,00 |
| Comp2_1 | M | 95,29 | 96,84 | 98,71 | 100,00 | 100,00 | 100,00 | 100,00 | 100,00 | 100,00 |
| Comp2_2 | F | 15,71 | 15,81 | 14,74 | 100,00 | 100,00 | 17,82 | 82,11 | 32,74 | 100,00 |
| Comp2_3 | F | 36,01 | 46,86 | 33,51 | 100,00 | 100,00 | 34,07 | 100,00 | 42,12 | 100,00 |
| Comp2_4 | F | 30,10 | 32,89 | 26,44 | 100,00 | 100,00 | 32,41 | 100,00 | 35,09 | 100,00 |
| Comp2_5 | M | 94,52 | 95,22 | 100,00 | 100,00 | 100,00 | 100,00 | 100,00 | 88,40 | 100,00 |
| Comp2_6 | M | 97,44 | 97,62 | 98,20 | 100,00 | 100,00 | 100,00 | 100,00 | 94,92 | 100,00 |
| Overall | Male | 92,19 | 93,05 | 94,71 | 100,00 | 100,00 | 90,89 | 96,39 | 91,42 | 100,00 |
| Overall | Female | 30,13 | 30,28 | 13,07 | 96,33 | 87,28 | 18,54 | 83,34 | 36,17 | 95,69 |

*F: Female, M: Male

In addition, some of the other SBEs also show this pattern. Typing of the eight SBEs on the complete F2 population will reveal whether these SNPs are also or more associated with the growth and meat traits.

**Conclusion**

In this study we have identified 23 SNPs in the turkey GDF8 gene. For two SNPs an association study on 1400 animals and several growth and meat traits was performed. A significant association is detected between SBE1 and several growth traits in a cross between two commercial lines that differ enormously in several growth traits. Animals that are homozygous G for SBE1 have approximately 400 grams more body weight at 120 days than animals that are homozygous A for SBE1. In total, this SNP (SBE1) explains 5.25 percent of the total variation in body weight in this cross. The frequencies of the G allele in several commercial lines are in line with the expectations based on the breeding goals that have been set in the last decades.

Eight more SBEs are being typed on the 1400 animals in the commercial cross at this moment.

## Claims

1. A method for selecting a bird of the species *Meleagris gallopavo* for having improved body weight gain characteristics, wherein said body weight gain is obtained by improved muscle deposition, said method comprising the steps of screening the genome of a population of candidate birds for the presence of at least one mutation in the myostatin gene associated with improved body weight gain characteristics.

2. Method according to claim 1, wherein said at least one mutation is a single nucleotide polymorphisms (SNP).

3. Method according to claim 2, wherein said at least one SNP is selected from the group consisting of SNPs numbered 1 to 23 as shown in Table 1.

4. Method according to any one of claims 1-3, wherein said body weight gain characteristics are higher growth rate and/or higher weight at market age.

5. Method according to any one of the preceding claims, wherein said screening comprises obtaining a nucleic acid sample from each bird of said population of candidate birds, and screening said nucleic acid sample for the presence of at least one SNP in the myostatin gene.

6. Method for producing a bird of the species *Meleagris gallopavo* having improved body weight gain characteristics, wherein said body weight gain is obtained by improved muscle deposition, said method comprising selecting a bird of the species *Meleagris gallopavo* according to a method of any one of claims 1-5, crossing said selected bird with a bird of a desirable turkey line and hatching the fertilized egg to produce the bird.

7. Method according to claim 6, wherein said desirable turkey line is an elite flock or a multiplication stock.

8. Method of producing a turkey line having improved body weight gain characteristics, wherein said body weight gain is obtained by improved muscle deposition, said method comprising increasing the number and/or frequency of mutations in the myostatin gene in said line, associated with improved body weight gain characteristics.

9. Method according to claim 8, wherein said mutations are SNPs selected from the group consisting of SNPs numbered 1 to 23 as shown in Table 1.

**10.** Method according to claim 8 or 9, wherein said body weight gain characteristics are higher growth rate and/or higher weight at market age.

**11.** Method according to any one of claims 8-10, optionally comprising the method of claim 6 or 7.

**12.** Method according to any one of claims 8-11, wherein said turkey line is an elite flock, a multiplication stock or a flock of commercials.

**13.** Method according to any one of claims 8-12, wherein said improved body weight gain characteristics are not associated with reduced egg production in female birds.

**14.** A bird of the species *Meleagris gallopavo* having improved body weight gain characteristics, wherein said body weight gain is obtained by improved muscle deposition, and wherein said bird has at least one, more preferably at least two, still more preferably at least 6 single nucleotide polymorphisms (SNP) in the myostatin gene associated with improved body weight gain characteristics.

**15.** Bird according to claim 14, wherein said at least one SNP is selected from the group consisting of SNPs numbered 1 to 23 as shown in Table 1.

**16.** Bird according to claim 14 or 15, wherein said body weight gain characteristics are higher growth rate and/or higher weight at market age.

**17.** Bird according to any one of claims 14-16, wherein said SNPs are silent mutations.

**18.** Bird according to any one of claims 14-17, wherein said body weight gain characteristics are not associated with reduced egg production in female birds.

**19.** A turkey line obtained by a method according to any one of claims 8-13.

**20.** A turkey line according to claim 19, wherein said turkey line is an elite flock, a multiplication stock or a flock of commercials.

**21.** A turkey line exhibiting a frequency of mutations in the myostatin gene of at least 80% for at least 1, preferably at least 2, more preferably at least 3, 4 or 5, even more preferably 6, 7 or 8, most preferably all of the SNPs of Table 1, wherein said frequency is expressed as the percentage of birds of said flock having the desired SNP.

## Figure 1

LOCUS Sequenced 5452 bp dna linear UNK
DEFINITION parts of turkey MSTN
ACCESSION unknown
FEATURES Location/Qualifiers
BASE COUNT 1698 a 981 c 933 g 1524 t 316 others
ORIGIN
1 gtgaaaaatg acacatggtc ctatttaaat aggcaaactg tgactttttg catgcaattc
61 tgggatttgt ttttgttcta acatatagac tcctcaattc cccaaatgaa aatgaatccc
121 atttttgtt tgtttgtttg ctagcagcat gtctagcatc atctacattm atctaarata
181 ttacaagcca tgacctgtaa ttatacaatg ctgtctaggt gtatstcatg cagaargtat
241 attaaaaccg taattttaaa aatataagtg ggatayagaa aacctacaga tgcttgaata
301 gaaakcaaag cacactatca cttcagttgg cataatacag aggtatactg aactgaaata
361 aaataggata tgaaaaatga agatttattg acagaaggaa tttaaacagt tyatgtaatt
421 tacaaatttc agtaacaatg gaaaaarcaa gctstgtaat attaraattg agtagctaat
481 gatattmctt aaaaatacct ttgctactga aggtctttca gtatttcatt tgctgcccag
541 gattttgctg acaaggcaaa cttcctttac ttaagaagtg tctacacact tcagtaattc
601 atttgatac taaagagtca aataattacc ccttasyttt agtcatacrt gtacaaaatc
661 ttttattcct gcttatctag catctatcag aaacacctat tacagttttc tgaaatttta
721 actgcttaat acacttaaac tgttgaacag cacagattcc taatgtttgc agtgtgttta
781 ttatgtactt **w**ttcctcttc tttacagaaa tccctcagaa ttttacttga gatagtacaa
841 accttcagtc aaaatagtga cagaatccta aagcaactct aaagaaagc**y** ctgcctcaat
901 tcatagtgca gctatgtgtt cagtgtttat ttaagaatga tagtgctgtc ttccagcact
961 gctgcccata gtacttggaa acatatcctt tcagtatgtg aagacgtatc ctctatgaaa
1021 ccaccagata aatcagttca cccttggctg taactaaatg ctgtctagtg acttgtgatc
1081 gacagggctt taacctctga cagctagatt cattgttggg acaacaacca atcgtcggtt
1141 ttgacgacat gagcctaatc aaagttggag tataaaagcc cccttgccat atataaggca
1201 caccagtgtg gcaagccgtc tctcagattg catttgcttt cacggatctg tttagaactg
1261 aaagaaaaag ggaaagaggg agaggggagg aaaaaaaaaa aaaaaaaaaa aaaagggaaa
1321 agctgcagtg actgtaagat catgcaaaag ctagcagtct atgtttatat ttacctgttc
1381 atgcagattt tagttcatcc ggtggctctt gatggcagta gtcagcccac agagaacgct
1441 gaaaaagacg gactgtgcaa tgcttg**y**acg tggagacaga atactaaatc ctccagaata
1501 gaagccataa aaattcaaat cctcagcaaa ctgcgcctgg aacaagcacc taacattagc
1561 agggacgtta ttaaacaact tttacccaaa gctcctccgc tgcaggaact gattgatcag
1621 tatgacgtcc agagagacga cagtagcgat ggctctttgg aagacgatga ctatcatgcc
1681 acaaccgaaa cgattatcac aatgcctacg gagtgtaagt aataaccctg ctgctttcgt
1741 ctcgcaccgc tcctccgaga gttgtttccg tgctgtagag ccacacaact ccttctcagg
1801 ctctcccaac aggctgctgg ct**s**gagtctg cggagggagg gaggagagtg tatttctaaa
1861 gaatttgagt caggttcaaa tgaccgtgtt gcgagccttt gttttactgt tcattt**k**ttc
1921 ggctccttgt aatgtgtgtg ccctgtagca cgcttaggat ttgcctaagt gtgcttagaa
1981 agcagaagga catctgcaca gttggctctg ttacgtgcag cattcaggat gtaccctatt
2041 gcagatagaa agtgcaagtg cagctaataa gataacttga ttttctgcat ggcattccac
2101 ccgctagtgt gtttgaaagc aatagaagtg agtagaaata cagcttagag taggagactt
2161 acctaactac agcatgttac agcaagtgta agcatagagt taaattcttg cttctttgct
2221 gatcccacag gctagcacca cccacctctg agttaaaact gtatacttga agcaaat**s**ta
2281 cgtgtaaccg atttaaat**r**t taaaatgtcc acattatagc tatcagctcc agtactcgtg
2341 ccaaggaaga atgaaggcat ttactgaatg agcagattct aagttatctt cttagtgcta
2401 aacatctgnn nnnnnnnnnn nnnnnnnnnn nnnnnnnnnn nnnnnnnnnn nnnnnnnnnn
2461 nnnnnnnnnn nnnnnnnnnn nnnnnnnnnn nnnnnnnnnn nnnnnnnnnn nnnnnnnnnn
2521 nnnnnnnnnn nnnaatattg taagacatcc tacatgatct agaaaaaaaa atgggtttat
2581 atatgcatat tactttttgt tccctgttca gtaatttgtt ctttccattc atttatagct
2641 gattttcttg tacaaatgga gggaaaacca aaatgttgct tctttaagtt tagctctaaa
2701 atacaatata acaaagtagt aaaggcacaa ttatggatat acttgaggca agtccaaaaa
2761 cctacaacgg tgtttgtgca gatcctgaga ctcattaaac ccatgaaaga cggtacaaga

Figure 1 (continued)

2821 tatactggaa ttcgatcttt gaaacttgac atgaacccag gcactggtat ctggcagagt
2881 attgatgtga agacagtgtt gcaaaattgg ctcaaacagc ctgaatccaa tttaggcatc
2941 gaaataaaag cttttgatga gaatggacga gatcttgctg taacattccc aggaccaggt
3001 gaagatggac tggtaagtct tttttaaaaa aatctcattt gaataccctg gatttttttt
3061 tttttttaa atcattgcaa agtgattcag ttgtctttta aggataaaaa tggaaatact
3121 gcttagcgaa tgtaagataa aggttctata tacccaattc tttcacactt tgctctcaat
3181 taatggcaaa atcattctga cattcccgtg cctccatttt cctgtttgaa aaattaagat
3241 gaattgcatt acctcagggt catgttgcaa ggcaccgact ggaaaacgtt acttaacaat
3301 ggatggtact atc**r**agtta cactatttcc aaacttgaga gcaaatactg cttaatgtga
3361 agatatttta cataaagaag ataaaagcaa ttcaactctt tctgtgataa gaaattgctc
3421 taagtagcat gaaaagattt ttcattcaac acataagaga atttctccct attctgtatg
3481 cgtttgtttt ccattaatga cagtgagaat tatctatgtt tatgaatcct aaattg**yr**ca
3541 tctt**yy**at**y**a ag**k**gacagaa tgattaattc ctcctactag aaatgtagga gtacataaat
3601 tctgcttgat acaataaatc catcataatg atgagaaagc ataaaataac ttgtggtttc
3661 tttctgca**w**g atgaacataa taaatctaaa gcataccagt ttaaccacag catttccctt
3721 gttgcttccc caaattgcta atctacaatc acagctgaac ttacc**r**aaac agctgaaaca
3781 actaagaaat ttctgctttg aataannnnn nnnnnnnnnn nnnnnnnnnn nnnnnnnnnn
3841 nnnnnnnnnn nnnnnnnnnn nnnnnnnnnn nnnnnnnnnn nnnnnnnnnn nnnnnnnnnn
3901 nnnnnnnnnn nnnnnnnnnn nnnnnnnnnn nnnnnnnnnn nnnnnnnnnn nnnnnnnnnn
3961 aatacatacg cctaccttgt taatacttcc gattcctttt cctcttctct cagaacccat
4021 ttttagaggt cagagttaca gacacaccaa aacggtcccg cagagatttt ggccttgact
4081 gcgacgagca ctcaacggaa tctcgatgtt gtcgctaccc gctgacagtg gattttgaag
4141 cttttggatg ggactggatt atagcaccta aaagatacaa agccaattac tgctctggag
4201 aatgtgaatt cgtatttcta cagaaatacc cgcacactca cctggtacac caagcaaatc
4261 caagaggctc agcaggccct tgctgcacac ccaccaagat gtcccctata aacatgctgt
4321 atttcaatgg aaaagaacaa ataatatatg gaaagatacc agccatggtt gtagatcgtt
4381 gcgggtgctc atgagaccat cgtgagatcc tccacctc**r**t aaattgtgga agccaccaaa
4441 aaaagctata tcccctcatc caacttatca actgtgaaat tacgtacgct aggcattgcc
4501 aacatccata tactgtacaa tcatacagac cacatatagc acaacacgag ctgcagaatg
4561 tgaactaaaa gacaatagaa ttacctaagg gctggctttg aaacaacgga caaagaagct
4621 acaatcaaaa taatgggatt taacaaatca gtttcttaca atgtgaggga atcaatattc
4681 agtcattcag a**y**acaaattt atacgcagtt ttcaacatat gttggtaatc aaaagtaagc
4741 tccttctcat ctgaggacag aaggagcagg cta**y**taaatc aacttcttca cagcttcact
4801 taatattgta tttacagaaa aatatatac**y** ggtaacatat acaccactac acaataccac
4861 cagaagcatc cttgaacact tgaatatata gtgcagagtt gtgataagat gaaattccat
4921 gtaaatggac aaatcctgaa gttagggatg gtatattgta tttagcgtgt ttccattcct
4981 ttttttcatt agtatgttag taatcaatgg caatggtgct acgtaagcag gctgagtaaa
5041 tagaaagata gttatctaag tgaaagaatt tggagtaata atgaatttgc cctatcctca
5101 ggtacactat tcaacattcg caagaaaaag ttattttta aaggaaggtg aacagttttc
5161 taaatgtagt aaaacaaaag gcagcacaga agtctaacgt tcaaaccata atcccatatt
5221 gtaatctgcc tttgcaacat caccgtttgc actatgataa ggccaatgca aatagttggt
5281 tgctacagac attgttaaa**r** aaacactttg atataactga cttgtgtaat atgtatgcat
5341 caatattttg taaataaatg tttatttttt aatcactgtt ggtatatgtt cattacgaaa
5401 agagt**k**atgt taatctgtac gttagtttaa taaacaaacc caggttttct ca
//

Figure 2

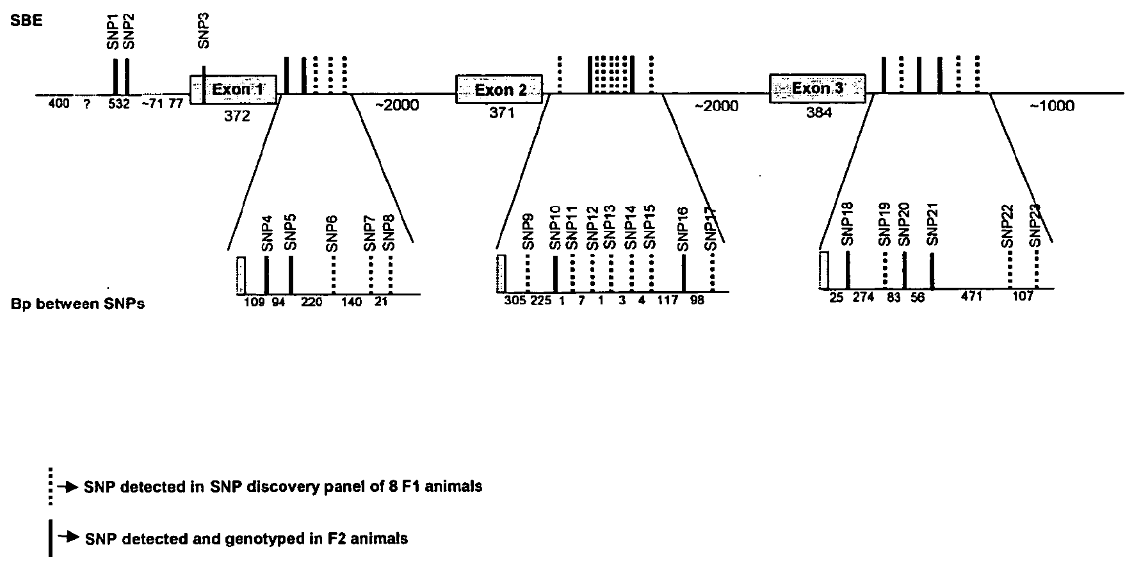
SBE
SNP1
SNP2
SNP3
Exon 1
Exon 2
Exon 3
400 ? 532 ~71 77
372
~2000
371
~2000
384
~1000
SNP4 SNP5 SNP6 SNP7 SNP8
SNP9 SNP10 SNP11 SNP12 SNP13 SNP14 SNP15 SNP16 SNP17
SNP18 SNP19 SNP20 SNP21 SNP22 SNP23
Bp between SNPs
109 94 220 140 21
305 225 1 7 1 3 4 117 98
25 274 83 56 471 107
SNP detected in SNP discovery panel of 8 F1 animals
SNP detected and genotyped in F2 animals

# Figure 3

```
LOCUS       AF019625        1128 bp    mRNA    linear   VRT 21-NOV-1997
DEFINITION  Meleagris gallopavo myostatin (MSTN) mRNA, complete cds.
ACCESSION   AF019625
VERSION     AF019625.1  GI:2623577
KEYWORDS    .
SOURCE      Meleagris gallopavo (turkey)
  ORGANISM  Meleagris gallopavo
            Eukaryota; Metazoa; Chordata; Craniata; Vertebrata;
Euteleostomi;
            Archosauria; Aves; Neognathae; Galliformes; Phasianidae;
Meleagris.
REFERENCE   1  (bases 1 to 1128)
  AUTHORS   McPherron,A.C. and Lee,S.J.
  TITLE     Double muscling in cattle due to mutations in the myostatin
gene
  JOURNAL   Proc. Natl. Acad. Sci. U.S.A. 94 (23), 12457-12461 (1997)
```

```
   1 atgcaaaagc tagcagtcta tgtttatatt tacctgttca tgcagatttt agttcatccg
  61 gtggctcttg atggcagtag tcagcccaca gagaacgctg aaaaagacgg actgtgcaat
 121 gcttgcacgt ggagacagaa tactaaatcc tccagaatag aagccataaa aattcaaatc
 181 ctcagcaaac tgcgcctgga acaagcacct aacattagca gggacgttat taaacaactt
 241 ttacccaaag ctcctccgct gcaggaactg attgatcagt atgacgtcca gagagacgac
 301 agtagcgatg gctctttgga agacgatgac tatcatgcca caaccgaaac gattatcaca
 361 atgcctacgg agtctgattt tcttgtacaa atggagggaa aaccaaaatg ttgcttcttt
 421 aagtttagct ctaaaataca atataacaaa gtagtaaagg cacaattatg gatatacttg
 481 aggcaagtcc aaaaacctac aacggtgttt gtgcagatcc tgagactcat taaacccatg
 541 aaagacggta caagatatac tggaattcga tctttgaaac ttgacatgaa cccaggcact
 601 ggtatctggc agagtattga tgtgaagaca gtgttgcaaa attggctcaa acagcctgaa
 661 tccaatttag gcatcgaaat aaaagctttt gatgagaatg gacgagatct tgctgtaaca
 721 ttcccaggac caggtgaaga tggactgaac ccatttttag aggtcagagt tacagacaca
 781 ccaaaacggt cccgcagaga ttttggcctt gactgcgacg agcactcaac ggaatctcga
 841 tgttgtcgct acccgctgac agtggatttt gaagcttttg gatgggactg gattatagca
 901 cctaaaagat acaaagccaa ttactgctct ggagaatgtg aattcgtatt tctacagaaa
 961 tacccgcaca ctcacctggt acaccaagca aatccaagag gctcagcagg cccttgctgc
1021 acacccacca agatgtcccc tataaacatg ctgtatttca atggaaaaga acaaataata
1081 tatggaaaga taccagccat ggttgtagat cgttgcgggt gctcatga
```

European Patent Office

# EUROPEAN SEARCH REPORT

Application Number
EP 05 07 7921

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/094315 A (REGENTS OF THE UNIVERSITY OF MINNESOTA; EL HALAWANI, MOHAMED, E; YOU,) 28 November 2002 (2002-11-28) | 14-21 | INV. C12Q1/68 |
| A | * the whole document * ----- | 1-13 | |
| X | WO 99/24618 A (THE JOHNS HOPKINS UNIVERSITY SCHOOL OF MEDICINE) 20 May 1999 (1999-05-20) | 14-21 | |
| A | * the whole document * ----- | 1-13 | |
| A | WO 98/33887 A (THE JOHNS HOPKINS UNIVERSITY SCHOOL OF MEDICINE) 6 August 1998 (1998-08-06) * the whole document * ----- | 1-13 | |

TECHNICAL FIELDS SEARCHED (IPC)
C12Q

The present search report has been drawn up for all claims

| Place of search | Date of completion of the search | Examiner |
|---|---|---|
| The Hague | 4 July 2006 | Gabriels, J |

4

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 05 07 7921

This annex lists the patent family members relating to the patent documents cited in the above-mentioned European search report.
The members are as contained in the European Patent Office EDP file on
The European Patent Office is in no way liable for these particulars which are merely given for the purpose of information.

04-07-2006

| Patent document cited in search report | | Publication date | Patent family member(s) | | | Publication date |
|---|---|---|---|---|---|---|
| WO 02094315 | A | 28-11-2002 | CA | 2433644 | A1 | 28-11-2002 |
| | | | EP | 1387695 | A2 | 11-02-2004 |
| | | | US | 2006088543 | A1 | 27-04-2006 |
| | | | US | 2002127234 | A1 | 12-09-2002 |
| WO 9924618 | A | 20-05-1999 | AU | 1390999 | A | 31-05-1999 |
| WO 9833887 | A | 06-08-1998 | AU | 6274298 | A | 25-08-1998 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4683195 A, Mullis
- US 4683202 A
- US 4800159 A
- EP 320308 A
- US 5270184 A
- US 5455166 A
- US 4458066 A
- WO 9302216 A


### Non-patent literature cited in the description


- **ZHILIANG G ; DAHAI Z ; NING L ; HUI L ; XUEMEI D ; CHANGXIN W.** *Sci China C Life Sci,* 04 February 2004, vol. 7 (1), 25-30
- **MEUWISSEN THE ; GODDARD ME.** Prediction of identity by descent probabilities from marker-haplotypes. *Genet. Sel. E,* 2001, vol. 33, 605-634
- **GILMOUR, A.R. ; CULLIS, B.S. ; WELHAM, S.J. ; THOMPSON, R.** *NSW Agriculture, Orange Agric. Inst. Orange,* 2000
- **BARANY.** *Proc. Natl. Acad. Sci. USA,* 1991, vol. 88, 189-193
- **GUATELLI et al.** *Proc. Natl. Acad. Sci. USA,* 1990, vol. 87, 1874-1878
- **KWOH et al.** *Proc. Natl. Acad. Sci. USA,* vol. 86, 1173-1177
- **LIZARDI et al.** *Bio/Technology,* 1988
- **L. MALEK et al.** Meth. Molec. Biol. Humana Press, Inc, 1994, vol. 28
- **CHEE et al.** *Science,* 1996, vol. 274 (5287), 610-614
- **SAMBROOK, J. ; E.F. FRITSCH ; T. MANIATIS.** Molecular Cloning: a Laboratory Manual. Cold Spring Harbor Laboratory Press, 2000
- **WATSON et al.** Recombinant DNA. Scientific American Books, 1992
- **AUSUBEL et al.** Current Protocols in Molecular Biology. Greene Publishing Associates, Inc. and John Wiley and Sons, Inc, 1998
- Current Protocols in Molecular Biology. 1995
- **SAMBROOK J ; RUSSELL DW.** Molecular Cloning: A Laboratory Manual. Cold Spring Harbor Laboratory Press, 2001
- **STADEN, R.** The Staden Sequence Analysis Package. *Molecular Biotechnology,* 1996, vol. 5, 233-241
- **BONFIELD JK ; STADEN R.** Experiment files and their application during large-scale sequencing projects. *DNA Sequence,* 1996, vol. 6, 109-117, http://www.mrc-lmb.cam.ac.uk/pubseq
- **EWING B ; HILLIER L ; WENDL MC ; GREEN P.** Base-calling of automated sequencer traces using Phred. I. Accuracy assessment. *Genome Res.,* 1998, vol. 8 (3), 175-185
- **BONFIELD, J.K. ; K. SMITH ; R. STADEN.** A new DNA sequence assembly program. *Nucleic Acids Res.,* 1995, vol. 24, 4992-4999
- **MEUWISSEN THE ; KARLSEN A ; LIEN S ; OLSAKER I ; GODDARD ME.** Fine Mapping of a Quantitative Trait Locus for Twinning Rate Using Combined Linkage and Linkage Disequilibrium Mapping. *Genetics,* 2002, vol. 161, 373-379

Annex to the application documents - subsequently filed sequences listing

SEQUENCE LISTING

<110> Nutreco Nederland B.V.

<120> Methods for improving turkey meat production

<130> P75088EP00

<140> EP 05077921.4
<141> 2005-12-19

<160> 30

<170> PatentIn version 3.3

<210> 1
<211> 5452
<212> DNA
<213> Meleagris gallopavo

<220>
<221> misc_feature
<222> (2409)..(2533)
<223> n is a, c, g, or t

<220>
<221> misc_feature
<222> (3806)..(3960)
<223> n is a, c, g, or t

<400> 1
gtgaaaaatg acacatggtc ctatttaaat aggcaaactg tgactttttg catgcaattc

60

tgggatttgt ttttgttcta acatatagac tcctcaattc cccaaatgaa aatgaatccc

120

attttttgtt tgtttgtttg ctagcagcat gtctagcatc atctacattm atctaarata

180

ttacaagcca tgacctgtaa ttatacaatg ctgtctaggt gtatstcatg cagaargtat

240

attaaaaccg taattttaaa aatataagtg ggatayagaa aacctacaga tgcttgaata

300

gaaakcaaag cacactatca cttcagttgg cataatacag aggtatactg aactgaaata

360

aaataggata tgaaaaatga agatttattg acagaaggaa tttaaacagt tyatgtaatt

420

tacaaatttc agtaacaatg gaaaaarcaa gctstgtaat attaraattg agtagctaat

480

gatattmctt aaaaatacct ttgctactga aggtctttca gtatttcatt tgctgcccag

540

gattttgctg acaaggcaaa cttcctttac ttaagaagtg tctacacact tcagtaattc
600
attttgatac taaagagtca aataattacc ccttasyttt agtcatacrt gtacaaaatc
660
ttttattcct gcttatctag catctatcag aaacacctat tacagttttc tgaaatttta
720
actgcttaat acacttaaac tgttgaacag cacagattcc taatgtttgc agtgtgttta
780
ttatgtactt wttcctcttc tttacagaaa tccctcagaa ttttacttga gatagtacaa
840
accttcagtc aaaatagtga cagaatccta aagcaactct aaagaaagcy ctgcctcaat
900
tcatagtgca gctatgtgtt cagtgtttat ttaagaatga tagtgctgtc ttccagcact
960
gctgcccata gtacttggaa acatatcctt tcagtatgtg aagacgtatc ctctatgaaa
1020
ccaccagata aatcagttca cccttggctg taactaaatg ctgtctagtg acttgtgatc
1080
gacagggctt taacctctga cagctagatt cattgttggg acaacaacca atcgtcggtt
1140
ttgacgacat gagcctaatc aaagttggag tataaaagcc cccttgccat atataaggca
1200
caccagtgtg gcaagccgtc tctcagattg catttgcttt cacggatctg tttagaactg
1260
aaagaaaaag ggaaagaggg agaggggagg aaaaaaaaaa aaaaaaaaaa aaaagggaaa
1320
agctgcagtg actgtaagat catgcaaaag ctagcagtct atgtttatat ttacctgttc
1380
atgcagattt tagttcatcc ggtggctctt gatggcagta gtcagcccac agagaacgct
1440
gaaaaagacg gactgtgcaa tgcttgyacg tggagacaga atactaaatc ctccagaata
1500
gaagccataa aaattcaaat cctcagcaaa ctgcgcctgg aacaagcacc taacattagc
1560

agggacgtta ttaaacaact tttacccaaa gctcctccgc tgcaggaact gattgatcag
1620
tatgacgtcc agagagacga cagtagcgat ggctctttgg aagacgatga ctatcatgcc
1680
acaaccgaaa cgattatcac aatgcctacg gagtgtaagt aataaccctg ctgctttcgt
1740
ctcgcaccgc tcctccgaga gttgtttccg tgctgtagag ccacacaact ccttctcagg
1800
ctctcccaac aggctgctgg ctsgagtctg cggagggagg gaggagagtg tatttctaaa
1860
gaatttgagt caggttcaaa tgaccgtgtt gcgagccttt gttttactgt tcatttkttc
1920
ggctccttgt aatgtgtgtg ccctgtagca cgcttaggat ttgcctaagt gtgcttagaa
1980
agcagaagga catctgcaca gttggctctg ttacgtgcag cattcaggat gtaccctatt
2040
gcagatagaa agtgcaagtg cagctaataa gataacttga ttttctgcat ggcattccac
2100
ccgctagtgt gtttgaaagc aatagaagtg agtagaaata cagcttagag taggagactt
2160
acctaactac agcatgttac agcaagtgta agcatagagt taaattcttg cttctttgct
2220
gatcccacag gctagcacca cccacctctg agttaaaact gtatacttga agcaaatsta
2280
cgtgtaaccg atttaaatrt taaaatgtcc acattatagc tatcagctcc agtactcgtg
2340
ccaaggaaga atgaaggcat ttactgaatg agcagattct aagttatctt cttagtgcta
2400
aacatctgnn nnnnnnnnnn nnnnnnnnnn nnnnnnnnnn nnnnnnnnnn nnnnnnnnnn
2460
nnnnnnnnnn nnnnnnnnnn nnnnnnnnnn nnnnnnnnnn nnnnnnnnnn nnnnnnnnnn
2520
nnnnnnnnnn nnnaatattg taagacatcc tacatgatct agaaaaaaaa atgggtttat
2580

atatgcatat tactttttgt tccctgttca gtaatttgtt ctttccattc atttatagct 2640

gattttcttg tacaaatgga gggaaaacca aaatgttgct tctttaagtt tagctctaaa 2700

atacaatata acaaagtagt aaaggcacaa ttatggatat acttgaggca agtccaaaaa 2760

cctacaacgg tgtttgtgca gatcctgaga ctcattaaac ccatgaaaga cggtacaaga 2820

tatactggaa ttcgatcttt gaaacttgac atgaacccag gcactggtat ctggcagagt 2880

attgatgtga agacagtgtt gcaaaattgg ctcaaacagc ctgaatccaa tttaggcatc 2940

gaaataaaag cttttgatga gaatggacga gatcttgctg taacattccc aggaccaggt 3000

gaagatggac tggtaagtct tttttaaaaa aatctcattt gaataccctg gatttttttt 3060

ttttttttaa atcattgcaa agtgattcag ttgtctttta aggataaaaa tggaaatact 3120

gcttagcgaa tgtaagataa aggttctata tacccaattc tttcacactt tgctctcaat 3180

taatggcaaa atcattctga cattcccgtg cctccatttt cctgtttgaa aaattaagat 3240

gaattgcatt acctcagggt catgttgcaa ggcaccgact ggaaaacgtt acttaacaat 3300

ggatggtact atcrgagtta cactatttcc aaacttgaga gcaaatactg cttaatgtga 3360

agatatttta cataaagaag ataaaagcaa ttcaactctt tctgtgataa gaaattgctc 3420

taagtagcat gaaaagattt ttcattcaac acataagaga atttctccct attctgtatg 3480

cgtttgtttt ccattaatga cagtgagaat tatctatgtt tatgaatcct aaattgyrca 3540

tcttyyatya agkgacagaa tgattaattc ctcctactag aaatgtagga gtacataaat 3600

tctgcttgat acaataaatc catcataatg atgagaaagc ataaaataac ttgtggtttc
3660
tttctgcawg atgaacataa taaatctaaa gcataccagt ttaaccacag catttccctt
3720
gttgcttccc caaattgcta atctacaatc acagctgaac ttaccraaac agctgaaaca
3780
actaagaaat ttctgctttg aataannnnn nnnnnnnnnn nnnnnnnnnn nnnnnnnnnn
3840
nnnnnnnnnn nnnnnnnnnn nnnnnnnnnn nnnnnnnnnn nnnnnnnnnn nnnnnnnnnn
3900
nnnnnnnnnn nnnnnnnnnn nnnnnnnnnn nnnnnnnnnn nnnnnnnnnn nnnnnnnnnn
3960
aatacatacg cctaccttgt taatacttcc gattcctttt cctcttctct cagaacccat
4020
ttttagaggt cagagttaca gacacaccaa aacggtcccg cagagatttt ggccttgact
4080
gcgacgagca ctcaacggaa tctcgatgtt gtcgctaccc gctgacagtg gattttgaag
4140
cttttggatg ggactggatt atagcaccta aaagatacaa agccaattac tgctctggag
4200
aatgtgaatt cgtatttcta cagaaatacc cgcacactca cctggtacac caagcaaatc
4260
caagaggctc agcaggccct tgctgcacac ccaccaagat gtcccctata aacatgctgt
4320
atttcaatgg aaaagaacaa ataatatatg gaaagatacc agccatggtt gtagatcgtt
4380
gcgggtgctc atgagaccat cgtgagatcc tccacctcrt aaattgtgga agccaccaaa
4440
aaaagctata tcccctcatc caacttatca actgtgaaat tacgtacgct aggcattgcc
4500
aacatccata tactgtacaa tcatacagac cacatatagc acaacacgag ctgcagaatg
4560
tgaactaaaa gacaatagaa ttacctaagg gctggctttg aaacaacgga caaagaagct
4620

acaatcaaaa taatgggatt taacaaatca gtttcttaca atgtgaggga atcaatattc 4680

agtcattcag ayacaaattt atacgcagtt ttcaacatat gttggtaatc aaaagtaagc 4740

tccttctcat ctgaggacag aaggagcagg ctaytaaatc aacttcttca cagcttcact 4800

taatattgta tttacagaaa aatatatacy ggtaacatat acaccactac acaataccac 4860

cagaagcatc cttgaacact tgaatatata gtgcagagtt gtgataagat gaaattccat 4920

gtaaatggac aaatcctgaa gttagggatg gtatattgta tttagcgtgt ttccattcct 4980

ttttttcatt agtatgttag taatcaatgg caatggtgct acgtaagcag gctgagtaaa 5040

tagaaagata gttatctaag tgaaagaatt tggagtaata atgaatttgc cctatcctca 5100

ggtacactat tcaacattcg caagaaaaag ttatttttta aaggaaggtg aacagttttc 5160

taaatgtagt aaaacaaaag gcagcacaga agtctaacgt tcaaaccata atcccatatt 5220

gtaatctgcc tttgcaacat caccgtttgc actatgataa ggccaatgca aatagttggt 5280

tgctacagac attgttaaar aaacactttg atataactga cttgtgtaat atgtatgcat 5340

caatattttg taaataaatg tttatttttt aatcactgtt ggtatatgtt cattacgaaa 5400

agagtkatgt taatctgtac gttagtttaa taaacaaacc caggttttct ca 5452

<210> 2
<211> 1128
<212> DNA
<213> Meleagris gallopavo

<220>
<221> CDS
<222> (1)..(1128)

<400> 2

atg caa aag cta gca gtc tat gtt tat att tac ctg ttc atg cag att
48
Met Gln Lys Leu Ala Val Tyr Val Tyr Ile Tyr Leu Phe Met Gln Ile
1 5 10 15

tta gtt cat ccg gtg gct ctt gat ggc agt agt cag ccc aca gag aac
96
Leu Val His Pro Val Ala Leu Asp Gly Ser Ser Gln Pro Thr Glu Asn
20 25 30

gct gaa aaa gac gga ctg tgc aat gct tgc acg tgg aga cag aat act
144
Ala Glu Lys Asp Gly Leu Cys Asn Ala Cys Thr Trp Arg Gln Asn Thr
35 40 45

aaa tcc tcc aga ata gaa gcc ata aaa att caa atc ctc agc aaa ctg
192
Lys Ser Ser Arg Ile Glu Ala Ile Lys Ile Gln Ile Leu Ser Lys Leu
50 55 60

cgc ctg gaa caa gca cct aac att agc agg gac gtt att aaa caa ctt
240
Arg Leu Glu Gln Ala Pro Asn Ile Ser Arg Asp Val Ile Lys Gln Leu
65 70 75 80

tta ccc aaa gct cct ccg ctg cag gaa ctg att gat cag tat gac gtc
288
Leu Pro Lys Ala Pro Pro Leu Gln Glu Leu Ile Asp Gln Tyr Asp Val
85 90 95

cag aga gac gac agt agc gat ggc tct ttg gaa gac gat gac tat cat
336
Gln Arg Asp Asp Ser Ser Asp Gly Ser Leu Glu Asp Asp Asp Tyr His
100 105 110

gcc aca acc gaa acg att atc aca atg cct acg gag tct gat ttt ctt
384
Ala Thr Thr Glu Thr Ile Ile Thr Met Pro Thr Glu Ser Asp Phe Leu
115 120 125

gta caa atg gag gga aaa cca aaa tgt tgc ttc ttt aag ttt agc tct
432
Val Gln Met Glu Gly Lys Pro Lys Cys Cys Phe Phe Lys Phe Ser Ser
130 135 140

aaa ata caa tat aac aaa gta gta aag gca caa tta tgg ata tac ttg
480
Lys Ile Gln Tyr Asn Lys Val Val Lys Ala Gln Leu Trp Ile Tyr Leu
145 150 155 160

agg caa gtc caa aaa cct aca acg gtg ttt gtg cag atc ctg aga ctc
528
Arg Gln Val Gln Lys Pro Thr Thr Val Phe Val Gln Ile Leu Arg Leu
165 170 175

att aaa ccc atg aaa gac ggt aca aga tat act gga att cga tct ttg

```
576
Ile Lys Pro Met Lys Asp Gly Thr Arg Tyr Thr Gly Ile Arg Ser Leu
            180                 185                 190

aaa ctt gac atg aac cca ggc act ggt atc tgg cag agt att gat gtg

624
Lys Leu Asp Met Asn Pro Gly Thr Gly Ile Trp Gln Ser Ile Asp Val
        195                 200                 205

aag aca gtg ttg caa aat tgg ctc aaa cag cct gaa tcc aat tta ggc

672
Lys Thr Val Leu Gln Asn Trp Leu Lys Gln Pro Glu Ser Asn Leu Gly
    210                 215                 220

atc gaa ata aaa gct ttt gat gag aat gga cga gat ctt gct gta aca

720
Ile Glu Ile Lys Ala Phe Asp Glu Asn Gly Arg Asp Leu Ala Val Thr
225                 230                 235                 240

ttc cca gga cca ggt gaa gat gga ctg aac cca ttt tta gag gtc aga

768
Phe Pro Gly Pro Gly Glu Asp Gly Leu Asn Pro Phe Leu Glu Val Arg
                245                 250                 255

gtt aca gac aca cca aaa cgg tcc cgc aga gat ttt ggc ctt gac tgc

816
Val Thr Asp Thr Pro Lys Arg Ser Arg Arg Asp Phe Gly Leu Asp Cys
            260                 265                 270

gac gag cac tca acg gaa tct cga tgt tgt cgc tac ccg ctg aca gtg

864
Asp Glu His Ser Thr Glu Ser Arg Cys Cys Arg Tyr Pro Leu Thr Val
        275                 280                 285

gat ttt gaa gct ttt gga tgg gac tgg att ata gca cct aaa aga tac

912
Asp Phe Glu Ala Phe Gly Trp Asp Trp Ile Ile Ala Pro Lys Arg Tyr
    290                 295                 300

aaa gcc aat tac tgc tct gga gaa tgt gaa ttc gta ttt cta cag aaa

960
Lys Ala Asn Tyr Cys Ser Gly Glu Cys Glu Phe Val Phe Leu Gln Lys
305                 310                 315                 320

tac ccg cac act cac ctg gta cac caa gca aat cca aga ggc tca gca

1008
Tyr Pro His Thr His Leu Val His Gln Ala Asn Pro Arg Gly Ser Ala
                325                 330                 335

ggc cct tgc tgc aca ccc acc aag atg tcc cct ata aac atg ctg tat

1056
Gly Pro Cys Cys Thr Pro Thr Lys Met Ser Pro Ile Asn Met Leu Tyr
            340                 345                 350

ttc aat gga aaa gaa caa ata ata tat gga aag ata cca gcc atg gtt

1104
Phe Asn Gly Lys Glu Gln Ile Ile Tyr Gly Lys Ile Pro Ala Met Val
```

355 360 365

gta gat cgt tgc ggg tgc tca tga

1128
Val Asp Arg Cys Gly Cys Ser
370 375

<210> 3
<211> 375
<212> PRT
<213> Meleagris gallopavo

<400> 3

Met Gln Lys Leu Ala Val Tyr Val Tyr Ile Tyr Leu Phe Met Gln Ile
1 5 10 15

Leu Val His Pro Val Ala Leu Asp Gly Ser Ser Gln Pro Thr Glu Asn
20 25 30

Ala Glu Lys Asp Gly Leu Cys Asn Ala Cys Thr Trp Arg Gln Asn Thr
35 40 45

Lys Ser Ser Arg Ile Glu Ala Ile Lys Ile Gln Ile Leu Ser Lys Leu
50 55 60

Arg Leu Glu Gln Ala Pro Asn Ile Ser Arg Asp Val Ile Lys Gln Leu
65 70 75 80

Leu Pro Lys Ala Pro Pro Leu Gln Glu Leu Ile Asp Gln Tyr Asp Val
85 90 95

Gln Arg Asp Asp Ser Ser Asp Gly Ser Leu Glu Asp Asp Asp Tyr His
100 105 110

Ala Thr Thr Glu Thr Ile Ile Thr Met Pro Thr Glu Ser Asp Phe Leu
115 120 125

Val Gln Met Glu Gly Lys Pro Lys Cys Cys Phe Phe Lys Phe Ser Ser
130 135 140

Lys Ile Gln Tyr Asn Lys Val Val Lys Ala Gln Leu Trp Ile Tyr Leu
145 150 155 160

Arg Gln Val Gln Lys Pro Thr Thr Val Phe Val Gln Ile Leu Arg Leu
165 170 175

Ile Lys Pro Met Lys Asp Gly Thr Arg Tyr Thr Gly Ile Arg Ser Leu
180 185 190

Lys Leu Asp Met Asn Pro Gly Thr Gly Ile Trp Gln Ser Ile Asp Val
195 200 205

```
Lys Thr Val Leu Gln Asn Trp Leu Lys Gln Pro Glu Ser Asn Leu Gly
    210                 215                 220

Ile Glu Ile Lys Ala Phe Asp Glu Asn Gly Arg Asp Leu Ala Val Thr
225                 230                 235                 240

Phe Pro Gly Pro Gly Glu Asp Gly Leu Asn Pro Phe Leu Glu Val Arg
                245                 250                 255

Val Thr Asp Thr Pro Lys Arg Ser Arg Arg Asp Phe Gly Leu Asp Cys
            260                 265                 270

Asp Glu His Ser Thr Glu Ser Arg Cys Cys Arg Tyr Pro Leu Thr Val
        275                 280                 285

Asp Phe Glu Ala Phe Gly Trp Asp Trp Ile Ile Ala Pro Lys Arg Tyr
    290                 295                 300

Lys Ala Asn Tyr Cys Ser Gly Glu Cys Glu Phe Val Phe Leu Gln Lys
305                 310                 315                 320

Tyr Pro His Thr His Leu Val His Gln Ala Asn Pro Arg Gly Ser Ala
                325                 330                 335

Gly Pro Cys Cys Thr Pro Thr Lys Met Ser Pro Ile Asn Met Leu Tyr
            340                 345                 350

Phe Asn Gly Lys Glu Gln Ile Ile Tyr Gly Lys Ile Pro Ala Met Val
        355                 360                 365

Val Asp Arg Cys Gly Cys Ser
    370                 375
```

<210> 4
<211> 22
<212> DNA
<213> Artificial Sequence

<220>
<223> Primer

<400> 4
gcttaatgca cgtgaactgt tg

22

<210> 5
<211> 20
<212> DNA
<213> Artificial Sequence

<220>
<223> Primer

<400> 5
ctaaacagat ccgggacagc

20

<210> 6
<211> 20
<212> DNA
<213> Artificial Sequence

<220>
<223> Primer

<400> 6
gggctttaac ctctgacagc

20

<210> 7
<211> 20
<212> DNA
<213> Artificial Sequence

<220>
<223> Primer

<400> 7
gggagagcct gagaaggagt

20

<210> 8
<211> 20
<212> DNA
<213> Artificial Sequence

<220>
<223> Primer

<400> 8
tacccaaagc tcctccactg

20

<210> 9
<211> 25
<212> DNA
<213> Artificial Sequence

<220>
<223> Primer

<400> 9
agcagatgtt tagcactaag aagat

25

<210> 10
<211> 21
<212> DNA
<213> Artificial Sequence

<220>
<223> Primer

<400> 10
ttgcatccac tctgttacca a

21

<210> 11
<211> 22
<212> DNA
<213> Artificial Sequence

<220>
<223> Primer

<400> 11
cattctcgaa gcaatatttc ca

22

<210> 12
<211> 21
<212> DNA
<213> Artificial Sequence

<220>
<223> Primer

<400> 12
gaatccaatt taggcatcga a

21

<210> 13
<211> 23
<212> DNA
<213> Artificial Sequence

<220>
<223> Primer

<400> 13
cagaatgatc aattgtgtag cag

23

<210> 14
<211> 23
<212> DNA
<213> Artificial Sequence

<220>
<223> Primer

<400> 14
tcctcaaatt acatacgcct acc

23

<210> 15
<211> 19
<212> DNA
<213> Artificial Sequence

<220>

<223> Primer

<400> 15
gttggcaatg cctagcgta

19

<210> 16
<211> 19
<212> DNA
<213> Artificial Sequence

<220>
<223> Primer

<400> 16
tacgctaggc attgccaac

19

<210> 17
<211> 20
<212> DNA
<213> Artificial Sequence

<220>
<223> Primer

<400> 17
ctcagcctgc ttacgtagca

20

<210> 18
<211> 20
<212> DNA
<213> Artificial Sequence

<220>
<223> Primer

<400> 18
tgctacgtaa gcaggctgag

20

<210> 19
<211> 23
<212> DNA
<213> Artificial Sequence

<220>
<223> Primer

<400> 19
gagaaaacct gggtttgttt att

23

<210> 20
<211> 64
<212> DNA
<213> Artificial Sequence

<220>
<223> Primer

<400> 20
tttttttttt tttttttttt tttttttttt ttttttttga ggatttagta ttctgtctcc

60

acgt

64

<210> 21
<211> 69
<212> DNA
<213> Artificial Sequence

<220>
<223> Primer

<400> 21
tttttttttt tttttttttt tttttttttt tttttttttt tttttttca tcgtgagatc

60

ctccacctc

69

<210> 22
<211> 14
<212> DNA
<213> Primer Promoter SBE3

<400> 22
tttttttttt tttt

14

<210> 23
<211> 35
<212> DNA
<213> Artificial Sequence

<220>
<223> Primer

<400> 23
tttcctaatg tttgcagtgt gtttattatg tactt

35

<210> 24
<211> 54
<212> DNA
<213> Artificial Sequence

<220>
<223> Primer

<400> 24
tttttttttt tttttttttt tttgacagaa tcctaaagca actctaaaga aagc

54

<210> 25
<211> 63
<212> DNA
<213> Artificial Sequence

<220>
<223> Primer

<400> 25
tttttttttt tttttttttt tttttttttt ttttttgcg agcctttgtt ttactgttca

60

ttt

63

<210> 26
<211> 59
<212> DNA
<213> Artificial Sequence

<220>
<223> Primer

<400> 26
tttttttttt tttttttttt tttttttttt tttttttttt ttcctccctc cgcagactc

59

<210> 27
<211> 44
<212> DNA
<213> Artificial Sequence

<220>
<223> Primer

<400> 27
attaatgaca gtgagaatta tctatgttta tgaatcctaa attg

44

<210> 28
<211> 68
<212> DNA
<213> Artificial Sequence

<220>
<223> Primer

<400> 28
tttttttttt tttttttttt tttttttttg tggttaaact ggtatgcttt agatttatta

60

tgttcatc

68

<210> 29

```
<211>  29
<212>  DNA
<213>  Artificial Sequence

<220>
<223>  Primer

<400>  29
ttttttttta ggacagaagg agcaggcta

29


<210>  30
<211>  38
<212>  DNA
<213>  Artificial Sequence

<220>
<223>  Primer

<400>  30
ttttttttgt ggtattgtgt agtggtgtat atgttacc

38
```